# EUROPÄISCHE PATENTSCHRIFT

(11) **EP 1 458 410 B1**
(45) Veröffentlichungstag und Bekanntmachung des Hinweises auf die Patenterteilung: **02.01.2008**
(21) Anmeldenummer: 02795235.7
(22) Anmeldetag: 19.12.2002
(51) Int. Cl.: C12Q 1/68, C12N 15/10, A61P 35/04

(54) **Verfahren zur Herstellung einer mRNA Tumorantigen Bibliothek**
Process for preparation of a mRNA tumour antigen library
Procédé pour la préparation d'une bibliothèque d'ARNm d'antigènes tumorales

(30) Priorität: 19.12.2001 DE 10162480
(43) Veröffentlichungstag der Anmeldung: 22.09.2004
(60) Teilanmeldung: 07013494.5
(73) Patentinhaber: Curevac GmbH, 72076 Tübingen (DE)
(72) Erfinder: HOERR, Ingmar, 72070 Tübingen (DE); VON DER MÜLBE, Florian, 72070 Tübingen (DE); PASCOLO, Steve, 72072 Tübingen (DE)
(74) Vertreter: Graf von Stosch, Andreas
(86) Internationale Anmeldenummer: PCT/EP2002/014577
(87) Internationale Veröffentlichungsnummer: WO 2003/051401

(56) Entgegenhaltungen:
- EP-A- 1 083 232
- WO-A-90/11092
- WO-A-98/34640
- WO-A-99/14346
- WO-A-02/098443
- HOERR INGMAR ET AL: "In vivo application of RNA leads to induction of specific cytotoxic T lymphocytes and antibodies." EUROPEAN JOURNAL OF IMMUNOLOGY, Bd. 30, Nr. 1, Januar 2000 (2000-01), Seiten 1-7, XP002243972 ISSN: 0014-2980
- BOCZKOWSKI D ET AL: "DENDRITIC CELLS PULSED WITH RNA ARE POTENT ANTIGEN-PRESENTING CELLSIN VITRO AND IN VIVO" JOURNAL OF EXPERIMENTAL MEDICINE, TOKYO, JP, Bd. 184, Nr. 2, August 1996 (1996-08), Seiten 465-472, XP002921123 ISSN: 0022-1007
- HOLCIK MARTIN ET AL: "Four highly stable eukaryotic mRNAs assemble 3' untranslated region RNA-protein complexes sharing cis and trans components." PROCEEDINGS OF THE NATIONAL ACADEMY OF SCIENCES OF THE UNITED STATES, Bd. 94, Nr. 6, 1997, Seiten 2410-2414, XP002243974 1997 ISSN: 0027-8424

## Beschreibung

Die vorliegende Erfindung betrifft eine pharmazeutische Zusammensetzung, enthaltend mindestens eine mRNA, umfassend mindestens einen für mindestens ein Antigen aus einem Tumor codierenden Bereich, in Verbindung mit einem wässrigen Lösungsmittel und vorzugsweise einem Cytokin, bspw. GM-CSF, und ein Verfahren zur Herstellung der pharmazeutischen Zusammensetzung. Die erfindungsgemäße pharmazeutische Zusammensetzung dient insbesondere der Therapie und/oder Prophylaxe gegen Krebs.

Die Gentherapie und die genetische Vakzinierung sind molekularmedizinische Verfahren, deren Anwendung in der Therapie und Prävention von Erkrankungen erhebliche Auswirkungen auf die medizinische Praxis haben wird. Beide Verfahren beruhen auf der Einbringung von Nukleinsäuren in Zellen bzw. in Gewebe des Patienten sowie auf der anschließenden Verarbeitung der durch die eingebrachten Nukleinsäuren codierten Information, d.h. der Expression der erwünschten Polypeptide.

Die übliche Vorgehensweise bisheriger Verfahren der Gentherapie und der genetischen Vakzinierung ist die Verwendung von DNA, um die benötigte genetische Information in die Zelle einzuschleusen. In diesem Zusammenhang sind verschiedene Verfahren zur Einbringung von DNA in Zellen, wie bspw. Calciumphosphat-Transfektion, Polypren-Transfektion, Protoplasten-Fusion, Elektroporation, Mikroinjektion und Lipofektion, entwickelt worden, wobei sich insbesondere die Lipofektion als geeignetes Verfahren herausgestellt hat.

Ein weiteres Verfahren, das insbesondere bei genetischen Vakzinierungsverfahren vorgeschlagen wurde, ist die Verwendung von DNA-Viren als DNA-Vehikel. Derartige Viren haben den Vorteil, daß aufgrund ihrer infektiösen Eigenschaften eine sehr hohe Transfektionsrate zu erzielen ist Die verwendeten Viren werden genetisch verändert, so daß in der transfizierten Zelle keine funktionsfähigen infektiösen Partikel gebildet werden. Trotz dieser Vorsichtsmaßnahme kann jedoch aufgrund möglicher Rekombinationsereignisse ein gewisses Risiko der unkontrollierten Ausbreitung der eingebrachten gentherapeutisch wirksamen sowie viralen Gene nicht ausgeschlossen werden.

Üblicherweise wird die in die Zelle eingebrachte DNA in gewissem Ausmaß in das Genom der transfizierten Zelle integriert. Einerseits kann dieses Phänomen einen erwünschten Effekt ausüben, da hierdurch eine langandauernde Wirkung der eingebrachten DNA erzielt werden kann. Andererseits bringt die Integration in das Genom ein wesentliches Risiko der Gentherapie mit sich. So kann es bspw. zu einer Insertion der eingebrachten DNA in ein intaktes Gen kommen, was eine Mutation darstellt, welche die Funktion des endogenen Gens behindert oder gar vollkommen ausschaltet. Durch solche Integrationsereignisse können einerseits für die Zelle lebenswichtige Enzymsysteme ausgeschaltet werden, andererseits besteht auch die Gefahr einer Transformation der so veränderten Zelle in einen entarteten Zustand, falls durch die Integration der Fremd-DNA ein für die Regulation des Zellwachstums entscheidendes Gen verändert wird. Daher kann bei der Verwendung von DNA-Viren als Gentherapeutika und Vakzine ein Risiko der Krebsbildung nicht ausgeschlossen werden. In diesem Zusammenhang ist auch zu beachten, daß zur wirksamen Expression der in die Zelle eingebrachten Gene die entsprechenden DNA-Vehikel einen starken Promotor, bspw. den viralen CMV-Promotor, enthalten. Die Integration derartiger Promotoren in das Genom der behandelten Zelle kann zu unerwünschten Veränderungen der Regulierung der Genexpression in der Zelle führen.

Ein weiterer Nachteil der Verwendung von DNA als Gentherapeutika und Vakzine ist die Induktion pathogener Anti-DNA-Antikörper im Patienten unter Hervorrufung einer möglicherweise tödlichen Immunantwort.

Im Gegensatz zu DNA ist der Einsatz von RNA als Gentherapeutikum oder Vakzin als wesentlich sicherer einzustufen. Insbesondere bringt RNA nicht die Gefahr mit sich, stabil in das Genom der transfizierten Zelle integriert zu werden. Des weiteren sind keine viralen Sequenzen, wie Promotoren, zur wirksamen Transkription, erforderlich. Darüber hinaus wird RNA wesentlich einfacher *in vivo* abgebaut. Wohl aufgrund der gegenüber DNA relativ kurzen Halbwertszeit von RNA im Blutkreislauf sind bisher keine anti-RNA-Antikörper nachgewiesen worden. Daher kann RNA für molekularmedizinische Therapieverfahren als Molekül der Wahl angesehen werden.

Allerdings bedürfen auf RNA-Expressionssystemen beruhende medizinische Verfahren vor einer breiteren Anwendung noch der Lösung einiger grundsätzlicher Probleme. Eines der Probleme bei der Verwendung von RNA ist der sichere, Zell- bzw. Gewebe-spezifische effiziente Transfer der Nukleinsäure. Da sich RNA in Lösung normalerweise als sehr instabil erweist, konnte durch die herkömmlichen Verfahren, die bei DNA verwendet werden, RNA bisher nicht oder nur sehr ineffizient als Therapeutikum bzw. Impfstoff verwendet werden.

Für die Instabilität sind RNA-abbauende Enzyme, sog. RNAasen (Ribonucleasen), verantwortlich. Selbst kleinste Verunreinigungen von Ribonucleasen reichen aus, um RNA in Lösung vollständig abzubauen. Der natürliche Abbau von mRNA im Cytoplasma von Zellen ist sehr fein reguliert. Diesbezüglich sind mehrere Mechanismen bekannt. So ist für eine funktionale mRNA die endständige Struktur von entscheidender Bedeutung. Am 5'-Ende befindet sich die sogenannte "Cap-Struktur" (ein modifiziertes Guanosin-Nucleotid) und am 3'-Ende eine Abfolge von bis zu 200 Adenosin-Nucleotiden (der sog. Poly-A-Schwanz). Über diese Strukturen wird die RNA als mRNA erkannt und der Abbau reguliert. Darüber hinaus gibt es weitere Prozesse, die RNA stabilisieren bzw. destabilisieren. Viele diese Prozesse sind noch unbekannt, oftmals scheint jedoch eine Wechselwirkung zwischen der RNA und Proteinen dafür maßgeblich zu sein. Bspw. wurde kürzlich ein "mRNA-Surveillance-System" beschrieben (Hellerin und Parker, Annu. Rev. Genet. 1999, 33: 229 bis 260), bei dem durch bestimmte Feedback-Protein-Wechselwirkungen im Cytosol unvollständige oder Nonsense-mRNA erkannt und dem Abbau zugänglich gemacht wird, wobei ein Hauptteil dieser Prozesse durch Exonucleasen vollzogen wird.

Im Stand der Technik sind einige Maßnahmen vorgeschlagen worden, um die Stabilität von RNA zu erhöhen und dadurch ihren Einsatz als Gentherapeutikum bzw. RNA-Vakzine zu ermöglichen.

In EP-A-108232 werden mRNA Vakzine offenbart, kodierend für ein einzelnes Tumorantigen, ein Polyepitop von mehreren bekannten Tumorantigenen oder eine Kollektion von mRNAs, erhalten durch eine (un)fraktionierte cDNA Bibliothek von Tumorgewebe und reverser Transkription in mRNA. Die mRNA kann stabilisiert werden durch 5'-cap, polyA Schwanz, 5'/3' UTR von beta-Globin, Komplexierung mit Protamin (oder anderen polykationischen Peptiden), und die Addition von RNasin. Die mRNA kann weiter für ein Cytokin (GM-CSF) kodieren und einen IRES enthalten. Eine CTL sowie humorale Antwort wird ausgelöst.
mRNA-Vakzine integrieren nicht in das Genom des Patienten und sind daher vorteilhaft gegenüber DNA-Vakzinen. Adjuvanzien werden nicht benötigt.

WO 99/14346 beschreibt weitere Verfahren zur Stabilisierung von mRNA. Insbesondere werden Modifizierungen der mRNA vorgeschlagen, welche die mRNA-Spezies gegenüber dem Abbau von RNasen stabilisieren. Derartige Modifikationen betreffen einerseits die Stabilisierung durch Sequenzmodifikationen, insbesondere Verminderung des G und/oder U-Gehalts durch Baseneliminierung oder Basensubstitution. Andererseits werden chemische Modifikationen, insbesondere die Verwendung von Nucleotidanaloga, sowie 5'- und 3'- Blockierungsgruppen, eine erhöhte Länge des Poly-A-Schwanzes sowie die Komplexierung der mRNA mit stabilisierenden Mitteln und Kombinationen der genannten Maßnahmen vorgeschlagen.

In den US-Patenten US 5,580,859 und US 6,214,804 werden unter anderem im Rahmen der "transienten Gentherapie" (TGT) mRNA-Vakzine und -Therapeutika offenbart. Es werden verschiedene Maßnahmen zur Erhöhung der Translationseffizienz und der mRNA-Stabilität beschrieben, die sich vor allem auf die nicht-translatierten Sequenzbereiche beziehen.

Bieler und Wagner (in: Schleef (Hrsg.), Plasmids for Therapy and Vaccination, Kapitel 9, Seiten 147 bis 168, Wiley-VCH, Weinheim, 2001) berichten von der Verwendung synthetischer Gene im Zusammenhang mit gentherapeutischen Methoden unter Verwendung von DNA-Vakzinen und lentiveralen Vektoren. Es wird die Konstruktion eines synthetischen, von HIV-1 abgeleiteten *gag*-Gens beschrieben, bei welchem die Codons gegenüber der Wildtyp-Sequenz derart modifiziert wurden (alternative Codonverwendung, engl. "codon usage"), daß sie der Verwendung von Codons entsprach, die in hoch exprimierten Säugergenen zu finden ist. Dadurch wurde insbesondere der A/T-Gehalt gegenüber der Wildtyp-Sequenz vermindert. Die Autoren stellen insbesondere eine erhöhte Expressionsrate des synthetischen *gag-*Gens in transfizierten Zellen fest. Des weiteren wurde in Mäusen eine erhöhte Antikörperbildung gegen das *gag*-Protein bei mit dem synthetischen DNA-Konstrukt immunisierten Mäusen und auch eine verstärkte Cytokinfreisetzung *in vitro* bei transfizierten Milzzellen von Mäusen beobachtet Schließlich konnte eine Induzierung einer cytotoxischen Immunantwort in mit dem *gag*-Expressionsplasmid Immunisierten Mäusen festgestellt werden. Die Autoren dieses Artikels führen die verbesserten Eigenschaften ihres DNA-Vakzins im wesentlichen auf einen durch die optimierte Codooverwendung hervorgerufene Veränderung des nucleocytoplasmanschen Transports der vom DNA- Vakzin exprimierten mRNA zurück. Im Gegensatz dazu halten die Autoren die Auswirkung der veränderten Codonverwendung auf die Translationseffizienz für gering.

Der vorliegenden Erfindung liegt daher die Aufgabe zugrunde, ein neues System zur Gentherapie und genetischen Vakzinierung für Tumoren bereitzustellen, das die mit den Eigenschaften von DNA-Therapeutika und -Vakzinen verbundenen Nachteile überwindet.

Diese Aufgabe wird durch die in den Ansprüchen gekennzeichneten Ausführungsformen der vorliegenden Erfindung gelöst.

Insbesondere wird eine pharmazeutische Zusammensetzung bereitgestellt, enthaltend mindestens eine mRNA, umfassend mindestens einen für mindestens ein Antigen aus einem Tumor codierenden Bereich, in Verbindung mit einem wässerigen Lösungsmittel.

Der Begriff "Antigen aus einem Tumor" bedeutet erfindungsgemäß, dass das entsprechende Antigen in mit einem Tumor assozierten Zellen exprimiert wird. Daher sind erfindungsgemäß Antigene aus Tumoren insbesondere solche, die in den entarteten Zellen selbst produziert werden. Vorzugsweise handelt es sich dabei um auf der Oberfläche der Zellen lokalisierte Antigene. Des Weiteren sind die Antigene aus Tumoren aber auch solche, die in Zellen exprimiert werden, welche nicht selbst (oder ursprünglich nicht selbst) entartet sind (waren), jedoch mit dem in Rede stehenden Tumor assoziiert sind. Dazu gehören bspw. auch Antigene, die mit Tumor-versorgenden Gefäßen bzw. deren (Neu-)Bildung zusammenhängen, insbesondere solche Antigene, die mit der Neovaskularisierung oder Angiogenese assoziiert sind, bspw. Wachstumsfaktoren wie VEGF, bFGF, usw. Weiterhin umfassen derartige mit einem Tumor zusammenhängende Antigene solche aus Zellen des den Tumor einbettenden Gewebes. Zu nennen sind hier entsprechende Antigene von Bindegewebszellen, zB. Antigene der extrazellulären Matrix.

Erfindungsgemäß wird in der pharmazeutischen Zusammensetzung eine (oder mehrere) mRNAs zur Therapie bzw. Impfung, d.h. Vakzinierung, zur Behandlung oder Prävention (Prophylaxe) von Krebserkrankungen verwendet. Die Vakzinierung beruht auf der Einbringung eines Antigens (oder mehrerer Antigene) eines Tumors, im vorliegenden Fall der genetischen Information für das Antigen in Form der für das oder die Antigen(e) codierenden mRNA, in den Organismus, insbesondere in die Zelle. Die in der pharmazeutischen Zusammensetzung enthaltene mRNA wird in das (Tumor-)Antigen translatiert, d.h. das von der modifizierten mRNA codierte Polypeptid bzw. antigene Peptid wird exprimiert, wodurch eine gegen dieses Polypeptid bzw. antigene Peptid gerichtete Immunantwort stimuliert wird. Im vorliegenden Fall der Verwendung als genetische Vakzine zur Behandlung von Krebs wird daher die Immunantwort durch Einbringung der genetischen Information für Antigene aus einem Tumor, insbesondere Proteine, die ausschließlich auf Krebszellen exprimiert werden, erreicht, in dem eine erfindungsgemäße pharmazeutische Zusammensetzung verabreicht wird, die eine für ein derartiges Krebsantigen codierende mRNA enthält. Dadurch wird das oder die Krebsantigen(e) im Organismus exprimiert, wodurch eine Immunantwort hervorgerufen wird, die wirksam gegen die Krebszellen gerichtet ist.

In ihrer Verwendung als Vakzine kommt die erfindungsgemäße pharmazeutische Zusammensetzung insbesondere zur Behandlung von Krebserkrankungen (wobei die mRNA vorzugsweise für ein tumorspezifisches Oberflächenantigen (TSSA) codiert); bspw. zur Behandlung von malignem Melanom, Kolon-Karzinorn, Lymphomen, Sarkomen, kleinzelligem Lungenkarzinom, Blastomen usw., in Betracht. Spezifische Beispiele von Tumorantigenen sind u.a. 707-AP, AFP, ART-4, BAGE, β-Catenin/m, Bcr-abl, CAMEL, CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A*0201-R170I, HPV-E7, HSP70-2M, HAST-2, hTERT (oder hTRT), iCE, KIAA0205, LAGE, LDLR/FUT, MAGE, MART-1/Melan-A, MC1R, Myosin/m, MUCL, MUM-1, -2, -3, NA88-A, NY-ESO,-1, p190 minor bcr-abl, Pml/RARa, PRAME, PSA, PSM, RAGE, RU1 oder RU2, SAGE, SART-1 oder SART-3, TEL/AML1, TPI/m, TRP-1, TRP-2, TRP-2/INT2 und WT1.

Gemäß einer weiteren bevorzugten Ausführungsform ist das oder sind die Antigen(e) aus einem Tumor ein Polyepitop des/der Antigens/Antigene aus einem Tumor. Ein "Polyepitop" eines Antigens bzw mehrerer Antigene ist eine Aminosäuresequenz, in der mehrere oder viele Regionen des/der Antigens/Antigene repräsentiert sind, die mit dem Antigenbindenden Teil eines Antikörpers oder mit einem T-Zell-Rezeptor in Wechselwirkung treten. Das Polyepitop kann dabei vollständig und unmodifiziert vorliegen. Es kann jedoch gemäß der vorliegenden Erfindung, insbesondere zur Optimierung der Antikörper/Antigen- bzw. T-Zell-Rezeptor/Antigen-Wechselwirkung, auch modifiziert vorliegen. Eine Modifikation gegenüber dem Wildtyp-Polyepitop kann bspw. eine Deletion, Addition und/oder Substitution eines oder mehrerer Aminosäurereste umfassen. Dementsprechend wird/werden in der für das modifizierte Polyepitop codierenden mRNA der vorliegenden Erfindung gegenüber der für das Wildtyp-Polyepitop codierenden mRNA ein oder mehrere Nucleotide entfernt, hinzugefügt und/oder ersetzt.

Um die Stabilität der in der pharmazeutischen Zusammensetzung der vorliegenden Erfindung enthaltene (m)RNA zu erhöhen, weist vorzugsweise jede in der pharmazeutischen Zusammensetzung enthaltene (m)RNA eine oder mehrere Modifikationen, insbesondere chemische Modifikationen, auf, welche zur Erhöhung der Halbwertszeit der (m)RNA (eine oder mehrere) im Organismus beitragen bzw. den Transfer der (m)RNA (eine oder mehrere) in die Zelle verbessern.

Beispielsweise gibt es in den Sequenzen eukaryotischer mRNAs destabilisierende Sequenzelemente (DSE), an welche Signalproteine binden und den enzymatischen Abbau der mRNA *in vivo* regulieren. Daher werden zur weiteren Stabilisierung der in der erfindungsgemäßen pharmazeutischen Zusammensetzung bevorzugt enthaltenen modifizierten mRNA gegebenenfalls im für das mindestens eine Antigen aus einem Tumor codierenden Bereich ein oder mehrere Veränderungen gegenüber dem entsprechenden Bereich der Wildtyp-mRNA vorgenommen, so daß keine destabilisierenden Sequenzelemente enthalten sind. Selbstverständlich ist es erfindungsgemäß ebenfalls bevorzugt, gegebenenfalls in den nicht-translatierten Bereichen (3'- und/oder 5'-UTR) vorhandene DSE aus der mRNA zu eliminieren.

Derartige destabilisierende Sequenzen sind bspw. AU-reiche Sequenzen ("AURES"), die in 3'-UTR-Abschnitten zahlreicher instabiler mRNA vorkommen (Caput et aL, Proc. Natl. Acad. Sci. USA 1986, 83: 1670 bis 1674). Die in der erfindungsgemäßen pharmazeutischen Zusammensetzung enthaltenen RNA-Moleküle sind daher vorzugsweise derart gegenüber der Wildtyp-mRNA verändert, daß sie keine derartigen destabilisierenden Sequenzen aufweisen. Dies gilt auch für solche Sequenzmotive, die von möglichen Endonucleasen erkannt werden, bspw. die Sequenz GAACAAG, die im 3' UTR-Segment des für den Transferin-Rezeptor codierenden Gens enthalten ist (Binder et aL, EMBO J. 1994, 13:1969 bis 1980). Auch diese Sequenzmotive werden bevorzugt in der modifizierten mRNA der erfindungsgemäßen pharmazeutischen Zusammensetzung eliminiert.

Einem Fachmann sind verschiedene Verfahren geläufig, die zur Substitution von Codons in der erfindungsgemäßen modifizierten mRNA geeignet sind. Im Falle kürzerer codierender Bereiche (die für biologisch wirksame oder antigene Peptide codieren) kann bspw. die gesamte mRNA chemisch unter Verwendung von Standartechniken synthetisiert werden.

Bevorzugt werden allerdings Basensubstitutionen unter Verwendung einer DNA-Matrize zur Herstellung der modifizierten mRNA mit Hilfe von Techniken der gängigen zielgerichteten Mutagenese eingeführt; Maniatis et al., Molecular Cloning: A Laboratory Manual, Cold Spring Harbor Laboratory Press, 3. Aufl., Cold Spring Harbor, NY, 2001.

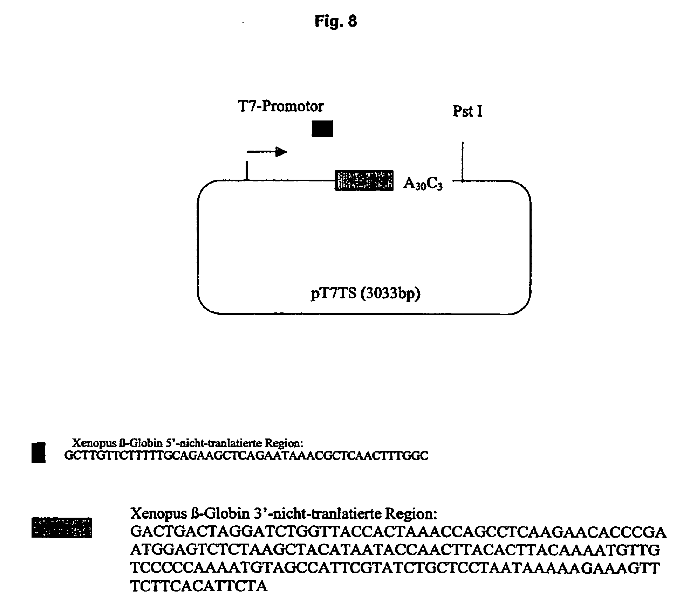

Bei diesem Verfahren wird zur Herstellung der mRNA daher ein entsprechendes DNA-Molekül *in vitro* transkribiert. Diese DNA-Matrize besitzt einen geeigneten Promotor, bspw. einen T7 -oder SP6-Promotor, für die *in vitro* Transkription, dem die gewünschte Nucleotidsequenz für die herzustellende mRNA und ein Termisationssignal für die in *in vitro* Transkribtion folgen. Erfindungsgemäß wird das DNA-Molekül, das die Matrize des herzustellenden RNA-Konstrukts bildet, durch fermentative Vermehrung und anschließende Isolierung als Teil eines in Bakterien replizierbaren Plasmids hergestellt. Als für die vorliegende Erfindung geeignete Plasmide können bspw. die Plasmide pT7TS (GenBank Zugriffsnummer U26404; Lai et aL, Development 1995, 121: 2349 bis 2360; vgl. auch Fig. 8), pGEM^{®}-Reihe, bspw. pGEM^{®}-1 (GenBank-Zugriffsnummer X65300; von Promega) und pSP64 (GenBank-Zugriffsnummer X65327) genannt werden; vgl. auch Mezei und Storts, Purification of PCR Products, in: Griffin und Griffin (Hrsg.), PCR Technology: Current Innovation, CRC Press, Boca Raton, FL, 2001.

Es kann so unter Verwendung kurzer synthetischer DNA-Oligonucleotide, die an den entstehenden Schnittstellen kurze einzelsträngige Übergänge aufweisen, oder durch chemische Synthese hergestellte Gene die gewünschte Nucleotidsequenz nach einem Fachmann geläufigen molekularbiologischen Methoden in ein geeignetes Plasmid cloniert werden (vgl. Maniatis et al., s.o.). Das DNA-Molekül wird dann aus dem Plasmid, in welchem es in einfacher oder mehrfacher Kopie vorliegen kann, durch Verdauung mit Restriktionsendonukleasen ausgeschnitten.

Die modifizierte mRNA, welche in der erfindungsgemäßen pharmazeutischen Zusammensetzung enthalten ist, kann darüber hinaus eine 5'-Cap-Struktur (ein modifiziertes Guanosin-Nucleotid) aufweisen. Als Beispiele von Cap-Strukturen können m7G(5')ppp (5'(A,G(5')ppp(5')A und G(5')ppp(5')G genannt werden.

Gemäß einer weiteren bevorzugten Ausführungsform der vorliegenden Erfindung enthält die modifizierte mRNA einen Poly(A⁺)-Schwanz von mindestens etwa 25, insbesondere mindestens etwa 30, vorzugsweise mindestens etwa 50 Nucleotiden, mehr bevorzugt mindestens etwa 70 Nucleotiden, besonders bevorzugt mindestens etwa 100 Nucleotiden. Der Poly(A⁺)-Schwanz kann jedoch auch 200 und mehr Nucleotide umfassen.

Für eine effiziente Translation der mRNA ist weiterhin eine wirksame Bindung der Ribosomen an die Ribosomen-Bindungsstelle (Kozak-Sequenz: GCCGCCACCAUGG, das AUG bildet das Startcodon) erforderlich. Diesbezüglich ist festgestellt worden, daß ein erhöhter A/U-Gehalt um diese Stelle herum eine effizientere Ribosomen-Bindung an die mRNA ermöglicht.

Des weiteren ist es möglich, in die mRNA eine oder mehrere sog. IRES (engl. "internal ribosomal entry side) einzufügen. Eine IRES kann so als alleinige Ribosomen-Bindungsstelle fungieren, sie kann jedoch auch zur Bereitstellung einer mRNA dienen, die mehrere Peptide bzw. Polypeptide codiert, die unabhängig voneinander durch die Ribosomen translatiert werden sollen ("multicistronische" oder "polycistronische" mRNA). Beispiele erfindungsgemäß verwendbarer IRES-Sequenzen sind diejenigen aus Picornaviren (z.B. FMDV), Pestviren (CFFV), Polioviren (PV), Enzephalo-Myocarditis-Viren (ECMV), Maul-und-Klauenseuche-Viren (FMDV), Hepatitis-C-Viren (HCV), Klassisches-Schweinefieber-Viren (CSFV), Murines-Leukoma-Virus (MLV), Simean-Immundefizienz-Viren (SIV) oder Cricket-Paralysis-Viren (CrPV).

Gemäß einer weiteren bevorzugten Ausführungsform der vorligenden Erfindung weist die mRNA in den 5'- und/oder 3'-nicht translatierten Bereichen Stabilisierungssequenzen auf, die befähigt sind, die Halbwertszeit der mRNA im Cytosol zu erhöhen.

Diese Stabilisierungssequenzen können eine 100%ige Sequenzhomologie zu natürlich vorkommenden Sequenzen, die in Viren, Bakterien und Eukaryoten auftreten, aufweisen, können aber auch teilweise oder vollständig synthetischer Natur sein. Als Beispiel für stabilisierende Sequenzen, die in der vorliegenden Erfindung verwendbar sind, können die nicht-translatierten Sequenzen (UTR) des β-Globingens, bspw. von *Homo sapiens* oder *Xenopus laevis,* genannt werden. Ein anderes Beispiel einer Stabilisierungssequenz weist die allgemeine Formel (C/U)CCANₓCCC(U/A)PyₓUC(C/U)CC auf, die im 3'UTR der sehr stabilen mRNA enthalten ist, die für a-Globin, a-(I)-Collagen, 15-Lipoxygenase oder für Tyrosia-Hydroxylase codiert (vgL Holcik et al., Proc. Natl. Acad. Sci. USA 1997, 94: 2410 bis 2414). Selbstverständlich können derartige Stabilisierungssequenzen einzeln oder in Kombination miteinander als auch in Kombination mit anderen, einem Fachmann bekannten Stabilisierungssequenzen verwendet werden.

Zur weiteren Stabilisierung der mRNA ist es außerdem bevorzugt, daß diese mindestens ein Analoges natürlich vorkommender Nucleotide aufweist. Dies beruht auf der Tatsache, daß die in den Zellen vorkommenden RNA-abbauenden Enzyme als Substrat vorzugsweise natürlich vorkommende Nucleotide erkennen. Durch Einfügen von Nucleotidanaloga kann daher der RNA-Abbau erschwert werden, wobei die Auswirkung auf die Translationseffizienz bei Einfügen von diesen Analoga, insbesondere in den codierenden Bereich der mRNA, einen positiven oder negativen Effekt auf die Translationseffizienz haben kann.

In einer keineswegs abschließenden Aufzählung können als Beispiele erfindungsgemäß verwendbarer Nucleotidanaloga Phosphoramidate, Phosphorthioate, Peptidnucleotide, Methylphosphonate, 7-Deazaguaonsin, 5-Methylcytosin und Inosin genannt werden. Die Herstellung derartiger Analoga sind einem Fachmann bspw. aus den US-Patenten 4,373,071, US 4,401,796, US 4,415,732, US 4,458,066, US 4,500,707, US 4,668,777, US 4,973,679, US 5,047,524, US 5,132,418, US 5,153,319, US 5,262,530 und 5,700,642 bekannt. Erfindungsgemäß können derartige Analoga in nicht-translatierten und translatierten Bereichen der modifizierten mRNA vorkommen.

Des weiteren kann der wirksame Transfer der, vorzugsweise modifizierten, mRNA in die zu behandelnden Zellen bzw den zu behandelnden Organismus dadurch verbessert werden, daß die mRNA mit einem kationischen oder polykationischen Agens, insbesondere einem entsprechenden Peptid oder Protein, assoziiert oder daran gebunden ist. Daher liegt die mRNA in der erfinungsgemäßen pharmazeutischen Zusammensetzung bevorzugt mit einem derartigen Agens komplexiert oder kondensiert vor. Insbesondere ist dabei die Verwendung von Protamin als polykationischem, Nucleinsäure-bindenden Protein besonders wirksam. Des weiteren ist die Verwendung anderer kationischer Peptide oder Proteine, wie Poly-L-Lysin, Poly-L-Arginin oder Histonen, ebenfalls möglich. Diese Vorgehensweise zur Stabilisierung der modifizierten mRNA ist in EP-A-1083232 beschrieben, deren diesbezüglicher Offenbarungsgehalt in die vorliegende Erfindung vollumfänglich eingeschlossen ist.

Darüber hinaus kann die erfindungsgemäß modifizierte mRNA neben dem antigenen oder dem gentherapeutisch wirksamen Peptid bzw. Polypeptid auch mindestens einen weiteren funktionalen Abschnitt enthalten, der bspw. für ein die Immunantwort förderndes Cytokin (Monokin, Lymphokin, Interleukin oder Chemokin, wie IL-1, IL-2, IL-3, IL-4, IL-5, IL-6, IL-7, IL-8, IL-9, IL-10, IL-12, IFN-α, IFN-γ, GM-CFS, LT-α oder Wachstumsfaktoren, wie hGH, codiert.

Des weiteren kann zur Erhöhung der Immunogenizität die erfindungsgemäße pharmazeutische Zusammensetzung ein oder mehrere Adjuvanzien enthalten. Unter "Adjuvans" ist dabei jede chemische oder biologische Verbindung zu verstehen, die eine spezifische Immunantwort begünstigt. In Abhängigkeit der verschiedenen Arten von Adjuvanzien können diesbezüglich verschiedene Mechanismen in Betracht kommen. Bspw. bilden Verbindungen, die eine Endocytose der in der pharmazeutischen Zusammensetzung enthaltenen modifizierten mRNA durch dendritische Zellen (DC) fördern, eine erste Klasse von verwendbaren Adjuvanzien. Andere Verbindungen, welche die Reifung der DC erlauben, bspw. Lipopolysaccharide, TNF-α oder CD4O-Ligand, sind eine weitere Klasse geeigneter Adjuvanzien. Allgemein kann jedes das Immunsystem beeinflussende Agens von der Art eines "Gefahrsignals" (LPS, GP96, Oligonucleotide mit dem CpG-Motiv) oder Cytokine, insbesondere GM-CFS, als Adjuvans verwendet werden, welche es erlauben, eine Immunantwort gegen ein Antigen, das durch die modifizierte mRNA codiert wird, zu erhöhen und/oder gerichtet zu beeinflussen. Insbesondere sind dabei die vorstehend genannten Cytokine bevorzugt. Weitere bekannte Adjuvanzien sind Aluminiumhydroxid, das Freud'sche Adjuvans sowie die vorstehend genannten stabilisierenden kationischen Peptide bzw. Polypeptide, wie Protamin. Des weiteren sind Lipopeptide, wie Pam3Cys, ebenfalls besonders geeignet, um als Adjuvanzien in der pharmazeutischen Zusammmensetzung der vorliegenden Erfindung eingesetzt zu werden; vgL Deres et al., Nature 1989, 342: 561-564.

Weitere besonders geeignete Adjuvanzien sind darüber hinaus (andere) RNA- oder auch mRNA-Spezies, die der pharmazeutischen Zusammensetzung der vorliegenden Erfindung zur Erhöhung der Immunogenizität zugesetzt werden können. Derartige Adjuvans-RNA ist vorteilhafter weise zur Stabilisation chemisch modifiziert ("cis-Modifikation" oder "cis-Stabilisierung") sein, beispielsweise durch die vorstehend genannten Nucleotidanaloga, insbesondere Phosphorthioat-modifizierte Nucleotide, oder aber durch die obigen weiteren Maßnahmen zur Stabilisation von RNA. Eine weitere vorteilhafte Möglichkeit der Stabilisation stellt die Komplexierung oder Assoziation ("trans-Assoziation" oder "trans-Modifikation" bzw. "trans-Stabilisierung") mit den vorstehend genannten kationischen oder polykationischen Agenzien, bspw. mit Protamin, dar.

Gemäß einer weiteren vorteilhaften Ausführungsform wird die Stabilität der in der pharmazeutischen Zusammensetzung enthaltenen RNA-Moleküle (mRNA, codierend für ein Tumorantigen, und ggf. Adjuvans-(m)RNA) durch einen oder mehrere RNase-Inhibitoren erhöht. Bevorzugte RNase-Inhibitoren sind Peptide oder Proteine, insbesondere solche aus Placenta (z.B. aus humaner Placenta) oder Pankreas. Derartige RNase-Inhibitoren können auch rekombinant vorliegen. Ein spezifisches Beispiel eines RNase-Inhibitors ist RNasin^{®}, der im Handel, z.B. bei Promega erhältlich ist. Derartige RNase-Inhibitoren sind allgemein zur Stabilisierung von RNA verwendbar. Daher wird erfindungsgemäß auch allgemein eine pharmazeutische Zusammensetzung bereitgestellt, enthaltend mindestens eine für mindestens ein Antigen codierende RNA, insbesondere mRNA, und mindestens ein wie vorstehend definierter RNase- Inhibitor, ggf. in Verbindung mit einem pharmazeutisch verträglichen Lösungsmittel, Träger und/oder Vehikel. Entsprechende Antigene in allgemeiner Form sowie Lösungsmittel, Träger bzw. Vehikel sind nachstehend definiert. Hinsichtlich bevorzugter Tumorantigene wird auf die diesbeszüglichen Ausführungen hinischtlich der bevorzugten pharmazeutischen Zusammensetzung, enthaltend mindestens eine für mindestens ein Antigen aus einem Tumor codierende mRNA, verwiesen.

Die erfindungsgemäße pharmazeutische Zusammensetzung enthält neben dem wässerigen Lösungsmittel und der mRNA vorzugsweise einen oder mehrere weitere(n) pharmazeutisch verträgliche(n) Träger und/oder ein oder mehrere weitere(s) pharmazeutisch verträgliche(s) Vehikel. Entsprechende Wege zur geeigneten Formulierung und Herstellung der erfindungsgemäßen pharmazeutischen Zusammensetzung sind bei "Remington's Pharmaceutical Sciences" (Mack Pub. Co., Easton, PA,1980) offenbart, das vollinhaltlich Bestandteil der Offenbarung der vorliegenden Erfindung ist Für die parenterale Verabreichung kommen als Trägerstoffe bspw neben sterilem Wasser oder sterilen Kochsalzlösungen als wässrigem Lösungsmittel auch Polyalkylenglykole, hydrogenierte Naphthalen und insbesondere biokompatible Lactidpolymere, Lactid/Glycolidcopolymer oder Polyoxyethylen/Polyoxypropylencopolymere in Betracht. Erfindungsgemäße Zusammensetzungen können Füllsubstanzen oder Substanzen, wie Lactose, Mannitol, Substanzen zur kovalenten Anknüpfung von Polymeren, wie z.B. Polyethylenglykol an erfindungsgemäße Inhibitoren, Komplexierung mit Metallionen oder Einschluß von Materialien in oder auf besondere Präparationen von Polymerverbindung, wie z.B. Polylactat, Polyglykolsäure, Hydrogel oder auf Liposomen, Mikroemulsion, Micellen, unilamellare oder multilamellare Vesikel, Erythrocyten-Fragmente oder Sphäroplasten, enthalten. Die jeweiligen Ausführungsformen der Zusammensetzungen werden abhängig vom physikalische Verhalten, beispielsweise in Hinblick auf die Löslichkeit, die Stabilität, Bioverfügbarkeit oder Abbaubarkeit gewählt. Kontrollierte oder konstante Freisetzung der erfindungsgemäßen Wukstoffkomponente in der Zusammensetzung schließt Formulierungen auf der Basis lipophiler Depots ein (z.B. Fettsäuren, Wachse oder Öle). Im Rahmen der vorliegenden Erfindung werden auch Beschichtungen erfindungsgemäßer Substanzen oder Zusammensetzungen, enthaltend solche Substanzen, nämlich Beschichtungen mit Polymeren offenbart (z.B. Polyoxamere oder Polyoxamine). Weiterhin können erfindungsgemäßen Substanzen bzw. Zusammensetzungen protektive Beschichtungen, z.B. Proteaseinhibitoren oder Permeabilitätsverstärker, aufweisen. Bevorzugte wässerige Trägermaterialien sind z.B. Wasser zur Injektion (WFI) oder Wasser, gepuffert mit Phosphat, Citrat oder Acetat usw., wobei der pH typischerweise auf 5,0 bis 8,0, vorzugsweise 6,0 bis 7,0, eingestellt wird. Das wässerige Lösungsmittel bzw. der oder die weitere(n) Träger bzw. das oder die weitere(n) Vehikel wird/werden zusätzlich vorzugsweise Salzbestandteile enthalten, z.B. Natriunchlorid, Kaliumchlorid oder andere Komponenten, welche die Lösung bspw. isotonisch machen. Weiterhin kann wässerige Lösungsmittel bzw. der oder die weitere(n) Träger bzw. das oder die weitere(n) Vehikel neben den vorstehend genannten Bestandteilen zusätzliche Komponenten, wie humanes Serumalbumin (HSA), Polysorbat 80, Zucker oder Aminosäuren, enthalten.

Die Art und Weise der Verabreichung und die Dosierung der erfindungsgemäßen pharmazeutischen Zusammensetzung hängen von der zu behandelnden Erkrankung und deren Fortsehrittsstadium, wie auch dem Körpergewicht, dem Alter und dem Geschlecht des Patienten ab.

Die Konzentration der modifizierten mRNA in derartigen Formulierungen kann daher innerhalb eines weiten Bereichs von 1 µg bis 100 mg/ml variieren. Die erfindungsgemäße pharmazeutische Zusammensetzung wird vorzugsweise parenteral, bspw. intravenös, intraarteriell, subkutan, intramuskulär, dem Patienten verabreicht. Ebenso ist es möglich, die pharmazeutische Zusammensetzung topisch oder oral zu verabreichen. Vorzugsweise wird die erfindungsgemäße pharmazeutische Zusammensetzung intradermal verabreicht. Weiterhin ist eine transdermale Applikation mit Hilfe von elektrischen Strömen bzw. durch osmotische Kräfte möglich. Des Weiteren kann die pharmazeutische Zusammensetzung der vorliegenden Erfindung lokal in einen Tumor injiziert werden.

Somit wird erfindungsgemäß auch ein Verfahren zur Behandlung bzw. ein Impfverfahren zur Prävention von Krebserkrankungen, bspw. der vorstehend genannten Erkrankungen, bereitgestellt, welches das Verabreichen der erfindungsgemäßen pharmazeutischen Zusammensetzung an einen Patienten, insbesondere einen Menschen, umfasst.

Gemäß einer bevorzugten Ausführungsform des Behandlungs- bzw. Impfverfahrens bzw. bei der vorstehend definierten Verwendung der erfindungsgemäßen mRNA, codierend für mindestens ein Antigen aus einem Tumor, zur Herstellung einer pharmazeutischen Zusammensetzung zur Behandlung und/oder Prävention von Krebserkrankungen wird dem Patienten neben der erfindungsgemäßen pharmazeutischen Zusammensetzung ein oder mehrere CytoKin(e) verabreicht.

Daher wird erfindungsgemäß auch allgemein ein Behandlungs- bzw. Impfverfahren bereitgestellt, umfassend das Verabreichen mindestens einer mindestens ein Antigen aus einem Tumor (gemäß obiger Definition) codierenden RNA, vorzugsweise mRNA, die ggf. gemäß den obigen Ausführungen stabilisiert ist (sind), und mindestens eines Cytokins, bspw. eines oder mehrerer der vorstehend genannten Cytokine, insbesondere GM-CSF, an einen Patienten, insbesondere einen Menschen. Das Verfahren dient insbesondere zur Behandlung und/oder Prävention entsprechender Krebserkrankungen (bspw. die obigen Krebserkrankungen). Dementsprechend ist die vorliegende Erfindung auch allgemein auf eine pharmazeutische Zusammensetzung gerichtet, umfassend mindestens eine mindestens ein Antigen aus einem Tumor (nach vorstehender Definition) codierende RNA, vorzugsweise mRNA, die ggf. gemäß den obigen Ausführungen stabilisiert ist (sind), und mindestens ein Cytokin, bspw. eines oder mehrerer der vorstehend genannten Cytokine, wie GM-CSF, vorzugsweise in Verbindung mit einem pharmazeutisch verträglichen Träger und/oder Vehikel, z.B. einem wässerigen Lösungsmittel, oder einem oder mehreren der vorstehend definierten Träger, Lösungsmittel bzw. Vehikel. Erfindungsgemäß wird somit auch die Verwendung von Cytokinen, bspw. eines oder mehrerer der vorstehend genannten Cytokine, insbesondere GM-CSF, in Kombination mit einem oder mehreren, wie vorstehend definierten RNA-Molekül(en), insbesondere mRNA, zur Behandlung und/oder Prävention von Krebserkrankungen (z.B. vorstehend angeführter Krebserkrankungen) offenbart.

Gemäß einer weiteren bevorzugten Ausführungsform der vorliegenden Erfindung wird das Cytokin, bspw. GM-CSF, gleichzeitig oder, was mehr bevorzugt ist, vor oder nach der pharmazeutischen Zusammensetzung, enthaltend die für mindestens ein Antigen aus einem Tumor codierende mRNA verabreicht (bzw. zur Herstellung eines entsprechendes Arzneimittels zur gleichzeitigen Verabreichung mit oder zur Verabreichung vor oder nach der vorstehend aufgeführten (m)RNA verwendet). Ganz besonders bevorzugt erfolgt die Verabreichung des Cytokins, insbesondere GM-CSF, kurz vor (z.B. etwa 15 min oder weniger, bspw. etwa 10 oder etwa 5 min) oder kürzere Zeit (bspw. etwa 5, 10, 15, 30, 45 oder 60 min) nach oder längere Zeit (z.B. etwa 2, 6, 12, 24 oder 36 h) nach der Verabreichung der vorstehend definierten pharmazeutischen Zusammensetzung bzw. allgemein nach der mindestens einen für mindestens ein Antigen aus einem Tumor codierenden (m)RNA.

Die Applikation des Cytokins, bspw. GM-CFS, kann dabei auf dem gleichen Wege wie die erfindungsgemäßen pharmazeutischen Zusammensetzungen bzw. die mindestens eine für mindestens ein Antigen aus einem Tumor codierende (m)RNA oder in einer davon getrennten Weise erfolgen. Geeignete Verabreichungswege sowie auch die geeigneten Formulierungsmöglichkeiten in Bezug auf das oder die Cytokin(e) können den obigen Ausführungen hinsichtlich der erfindungsgemäßen pharmazeutischen Zusammensetzungen entnommen werden. Bei einem humanen Patienten ist insbesondere eine GM-CFS-Dosis von 100 Mikrogramm/m² empfehlenswert. Besonders bevorzugt erfolgt die Verabreichung des Cytokins, bspw. GM-CFS, durch eine s.c.-Injektion.

Vorzugsweise werden die pharmazeutischen Zusammensetzungen der vorliegenden Erfindung bzw. die für ein Antigen aus einem Tumor codierende RNA und gegebenenfalls damit zusammenhängend das oder die Cytokin(e) in Form von Intervall-Dosen appliziert. Beispielsweise kann eine Dosis einer erfindungsgemäßen pharmazeutischen Zusammensetzung in kürzeren Intervallen, bspw. täglich, jeden zweiten Tag, jeden dritten Tag usw., oder aber, was mehr bevorzugt ist, in längeren Intervallen, bspw. einmal wöchentlich, einmal in zwei Wochen, einmal in drei Wochen, einmal im Monat usw. Dabei können die Intervalle auch veränderlich sein, wobei insbesondere die immunologischen Parameter des Patienten zu berücksichtigen sind. Bspw. kann die Verabreichung einer erfindungsgemäßen pharmazeutischen Zusammensetzung (und gegebenenfalls damit zusammenhängend auch die Verabreichung des oder der Cytokins/Cytokine) einem Behandlungsschema folgen, bei dem zu Beginn der Behandlung das Intervall kürzer ist, bspw. einmal in zwei Wochen, und dann, je nach Behandlungsverlauf bzw. den entsprechend bestimmten immunologischen Parametern des Patienzen, das Intervall auf bspw. einmal im Monat verlängert wird. Je nach Patient, insbesondere dessen Zustand und seinen immunologischen Parametern, kann so ein auf das jeweilige Individuum zugeschnittenes Therapieschema angewandt werden.

Ein weiterer Gegenstand der vorliegenden Anmeldung betrifft ein Verfahren zur Herstellung der vorstehend definierten pharmazeutischen Zusammensetzung, umfassend die Schritte:
(a) Herstellen einer cDNA-Bibliothek oder eines Teils davon aus Tumorgewerbe eines Patienten,
(b) Herstellen einer Matrize für die *In vitro* -Transkription von RNA anhand der cDNA-Bibliothek oder eines Teils davon und
(c) *In vitro*-Transkribieren der Matrize.

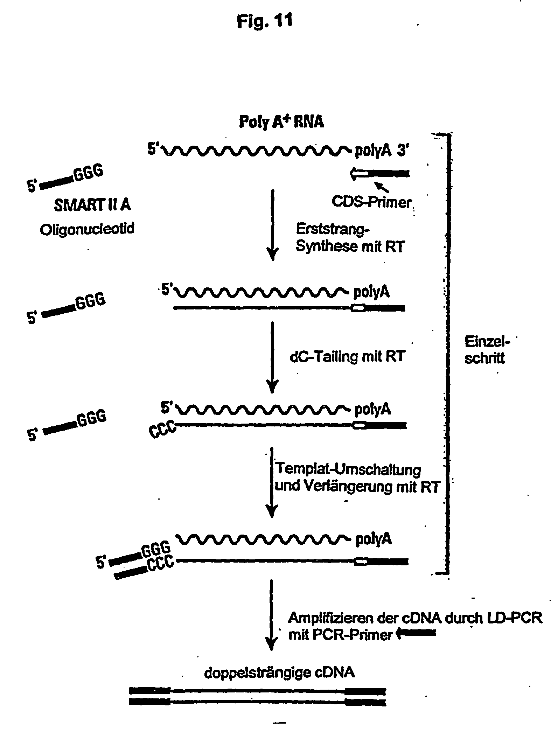

Das Tumorgewebe des Patienten kann bspw. durch eine einfache Biopsie gewonnen werden. Es kann aber auch durch operative Entfernung von Tumor-befallenem Gewebe bereitgestellt werden. Des Weiteren kann die Herstellung der cDNA-Bibliothek oder eines Teils davon gemäß Schritt (a) des Herstellungsverfahrens der vorliegenden Erfindung durchgeführt werden, nachdem das entsprechende Gewebe zur Lagerung, vorzugsweise auf Temperaturen unterhalb von -70 °C, tiefgefroren wurde. Zur Herstellung der cDNA-Bibliothek oder eines Teils davon wird zunächst eine Isolierung der Gesamt-RNA, bspw. aus einer Tumorgewebe-Biopsie, durchgeführt. Verfahren hierzu sind bspw. in Maniatis et aL, *supra,* beschrieben. Des Weiteren sind hierfür entsprechende Kits im Handel, bspw. bei der Fa. Roche AG (z.B. das Produkt "High Pure RNA Isolation Kit"), erhältlich. Aus der Gesamt-RNA wird gemäß einem Fachmann bekannter Verfahren (vgL bspw. Maniatis et aL, *supra*) die entsprechende Poly(A⁺)-RNA isoliert. Auch hierfür sind im Handel entsprechende Kits erhältlich. Ein Beispiel ist das "High Pure RNA Tissue Kit" der Fa. Roche AG. Ausgehend von der so gewonnenen Poly(A⁺)-RNA wird danach die cDNA-Bibliothek hergestellt (vgL auch hierzu bspw. Maniatis et al., *supra*). Auch für diesen Schritt bei der Herstellung der cDNA-Bibliothek stehen einem Fachmann im Handel erhältliche Kits, bspw. das "SMART PCR cDNA Synthesis Kit" der Fa. Clontech Inc., zur Verfügung. Die einzelnen Unterschritte von der Poly(A⁺)-RNA zur doppelsträngigen cDNA sind am Beispiel des Verfahrens gemäß dem "SMART PCR cDNA Synthesis Kit" der Fa. Clontech Inc. in der Fig. 11 schematisch dargestellt.

Gemäß Schritt (b) des vorstehenden Herstellungsverfahrens wird ausgehend von der cDNA Bibliothek (oder eines Teils davon) eine Matrize für die *In vitro* -Transkription synthetisiert. Dies erfolgt erfindungsgemäß insbesondere dadurch, daß die erhaltenen cDNA-Fragmente in einen geeigneten RNA-Produktionsvektor cloniert werden. Die geeignete DNA-Matrize und erfindungsgemäß bevorzugte Plasmide sind bereits vorstehend im Zusammenhang mit der Herstellung der mRNA für die erfindungsgemäße pharmazeutische Zusammensetzung angegeben.

Zur *In vitro* -Transkription der im erfindungsgemäßen Schritt (b) hergestellten Matrize wird diese, wenn sie als zirkuläre Plasmid-(c)DNA vorliegt, zunächst mit einem entsprechenden Restriktionsenzym linearisiert. Vorzugsweise wird das so geschnittene Konstrukt vor der eigentlichen *In vitro* -Transkription noch einmal gereinigt, bspw. durch entsprechende PhenolChloroform- und/oder Chloroform/Phenol/Isoamylalkohol-Gemische. Hierdurch wird insbesondere sichergestellt, daß die DNA-Matrize in proteinfreier Form vorliegt. Als nächstes erfolgt die enzymatische Synthese der RNA ausgehend von der gereinigten Matrize. Dieser Unterschritt erfolgt in einem entsprechenden Reaktionsgemisch, enthaltend die linearisierte, proteinfreie DNA-Matrize in einem geeigneten Puffer, dem vorzugsweise ein Ribonuclease-Inhibitor zugesetzt wird unter Verwendung eines Gemischs der benötigten Ribonucleotidtriphosphate (rATP, rCTP, rUTP und rGTP) und einer ausreichenden Menge einer RNA-Polymerase, bspw. T7-Polymerase. Das Reaktionsgemisch liegt dabei in RNase-freiem Wasser vor. Bevorzugt wird bei der eigentlichen enzymatischen Synthese der RNA auch ein CAP-Analogon zugefügt. Nach einer entsprechend langen, bspw. 2 h, Inkubation bei 37°C, wird die DNA-Matrize durch Zugabe von. RNase-freier DNase abgebaut, wobei bevorzugt wieder bei 37°C inkubiert wird.

Vorzugsweise wird die so hergestellte RNA mittels Ammoniumacetat/Ethanol gefällt und gegebenenfalls ein oder mehrmals mit RNase-freiem Ethanol gewaschen. Schließlich wird die so gereinigte RNA getrocknet und gemäß einer bevorzugten Ausführungsform in RNase-freiem Wasser aufgenommen. Des Weiteren kann die so hergestellte RNA mehreren Extraktionen mit Phenol/Chloroform bzw. Phenol/Chloroform/Isoamylalkohol unterworfen werden.

Gemäß einer weiteren bevorzugten Ausführungsform des vorstehend definierten Herstellungsverfahrens wird nur ein Teil einer Gesamt-cDNA-Bibliothek gewonnen und in entsprechende mRNA-Moleküle überführt. Daher kann erfindungsgemäß auch eine sog. Subtraktionsbibliothek ak Teil der Gesamt-cDNA-Bibliothek verwendet werden, um die erfindungsgemäßen mRNA-Moleküle bereitzustellen. Ein bevorzugter Teil der cDNA-Bibliothek des Tumorgewebes codiert für die tumorspezifischen Antigene. Bei bestimmten Tumoren sind die entsprechenden Antigene bekannt. Es kann gemäß einer weiteren bevorzugten Ausführungsform der für die tumorspezifischen Antigene codierende Teil der cDNA-Bibliothek zunächst ermittelt werden (d.h. vor Schritt (a) des vorstehend definierten Verfahrens). Dies erfolgt vorzugsweise dadurch, daß die Sequenzen der tumorspezifischen Antigene durch einen Abgleich mit einer entsprechenden cDNA-Bibliothek aus gesundem Gewebe festgestellt werden.

Der erfindungsgemäße Abgleich umfasst insbesondere einen Vergleich der Expressionsmuster des gesunden Gewebes mit dem des in Rede stehenden Tumorgewebes. Entsprechende Expressionsmuster werden können auf Nukleinsäureebene bspw. mit Hilfe geeigneter Hybridisierungsexperimente bestimmt. Hierzu können bspw. die entsprechenden (m)RNA- oder cDNA-Bibliotheken der Gewebe jeweils in geeigneten Agarose- oder Polyacrylamid-Genen aufgetrennt, auf Membranen überführt und mit entsprechenden Nukleinsäure-Sonden, vorzugsweise Oligonukleotid-Sonden, welche die jeweiligen Gene repräsentieren, hybridisiert werden (Northern- bzw. Southern-Blots). Ein Vergleich der entsprechenden Hybridisierungen liefert somit diejenigen Gene, die entweder ausschließlich vom Tumorgewebe oder darin stärker exprämiert werden.

Gemäß einer weiteren bevorzugten Ausführungsform werden die genannten Hybridisierungsexperimente mit Hilfe einer Diagnose durch Mikroarrays (ein oder mehrere Mikroarrays). Ein entsprechender DNA-Mikroarray umfaßt eine definierte Anordnung, insbesondere auf kleinem oder kleinstem Raum, von Nukleinsäure-, insbesondere Oligonukleotid-Sonden, wobei jede Sonde bspw. jeweils ein Gen, dessen Anwesenheit oder Abwesenheit in der entsprechenden (m)RNA- oder cDNA-Bibliothek zu untersuchen ist, repräsentiert. In einer entsprechenden Mikroanordnung können so Hunderte, Tausende und sogar Zehn- bis Hunderttasuende von Genen repräsentiert sein. Zur Analyse des Expressionsmusters des jeweiligen Gewebes wird dann entweder die Poly(A⁺)-RNA. oder, was bevorzugt ist, die entsprechende cDNA mit einem geeigneten Marker, insbesondere werden hierzu Fluoreszenzmarker verwendet, markiert und unter geeigneten Hybridisierungsbedingungen mit dem Mikroarray in Kontakt gebracht. Bindet eine cDNA-Spezies an einem auf dem Mikroarray vorhandenen Sondenmolekül, insbesondere einem Oligonukleotid-Sondenmolekül, wird dementsprechend ein mehr oder minder stark ausgeprägtes Fluoreszenzsignal, das mit einem geeigneten Detektionsgerät, bspw. einem entsprechend ausgelegten Fluroeszenzspektrometer, gemessen werden kann, beobachtet. Je stärker die cDNA (oder RNA) -Spezies in der Bibliothek repräsentiert ist, desto größer wird das Signal, bspw. das Fluoreszenzsignal, sein. Das entsprechende Mikroarray-Hybridisierungsexperiment (bzw. mehrere oder viele davon) wird (werden) getrennt für das Tumorgewebe und das gesunde Gewebe durchgeführt. Die Differenz der aus den Mikroarray-Experimenten ausgelesenen Signale läßt daher auf die ausschließlich oder vermehrt vom Tumorgewebe exprimierten Gene schließen. Derartige DNA-Mikroarray-Analysen sind bspw. in Schena (2002), Mikroarray Analysis, ISBN 0-471-41443-3, John Wiley & Sons, Inc., New York, dargestellt, wobei der diesbezügliche Offenbarungsgehalt dieser Druckschrift vollumfänglich in die vorliegende Erfindung aufgenommen ist

Die Erstellung tumorgewebsspezifischer Expressionsmuster ist jedoch keineswegs auf Analysen auf Nukleinsäureebene beschränkt. Einem Fachmann sind selbstverständlich auch im Stand der Technik bekannte Verfahren geläufig, welche der Expressionsanalyse auf Proteinebene dienen. Hier sind insbesondere Techniken der 2D-Gelelektrophorese und der Massensprektrometrie zu nennen, wobei diese Techniken vorteilhafterweise auch mit Proteinbiochips (also Mikroarrays auf Proteinebene, bei denen bspw. ein Proteinextrakt aus gesundem bzw. Tumorgewebe mit auf dem Mikroarray-Substrat aufgetragenen Antikörpern und/oder Peptiden in Kontakt gebracht wird), kombiniert werden können. Hinsichtlich der massenspektroskopischen Verfahren sind diesbezüglich insbesondere MALDI-TOF- ("matrix assisted laser desorption/ionisation-time of flight"-) Verfahren zu nennen. Die genannten Techniken zur proteinchemischen Analyse zur Gewinnung des Expressionsmusters von Tumor- im Vergleich zu gesundem Gewebe sind bspw. in Rehm (2000) Der Experimentator: Proteinbiochemie/Proteomics, spektrum Akademischer Verlag, Heidelberg, 3. Aufl., beschrieben, auf dessen diesebezüglichen Offenbarungsgehalt in der vorliegenden Erfindung *expressis* verbis ausdrücklich Bezug genommen. Hinsichtlich Protein-Mikroarrays wird außerdem wiederum auf die diesbszüglichen Ausführungen in Schena (2002), *supra,* verwiesen.

Die Figuren zeigen:

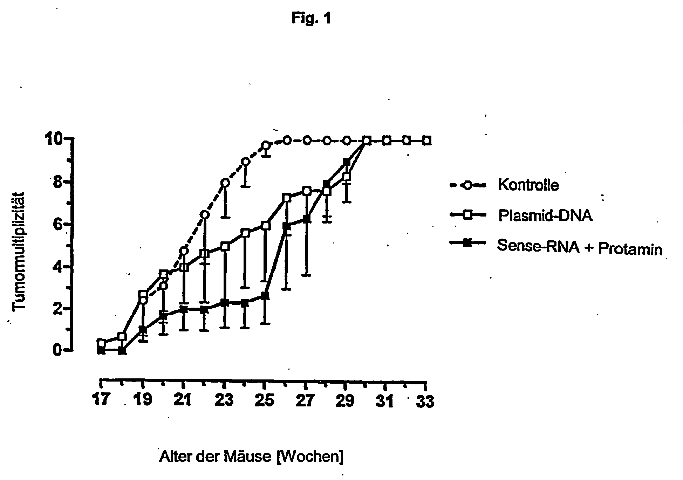

- Fig.1: zeigt in einer graphischen Darstellung die Ergebnisse einer Tumorvakzinierung von Mäusen (Ratten-Her-2/neu-transgene Tiere), die spontan Mammakarzinome entwickeln, mit RNA. Es ist die Tumormultiplizität auf der y-Achse gegen das Alter der Mäuse auf der x-Achse aufgetragen. Unbehandelte Mäuse (n = 4), die als Kontrolle dienten, wiesen alle in einem Alter von 6 Monaten Tumore auf. Drei Mäusen wurde für Her-2/neu codierende DNA injiziert, wobei eine Maus nach 10 Monaten tumorfrei war. Als weitere Negativkontrolle erhielten 4 Mäuse eine zur mRNA für Her-2/neu komlementäre Antisense-mRNA. Diese Mäuse wiesen ebenfalls nach 6 Monaten alle Tumore auf (nicht gezeigt). Im Gegensatz dazu war eine von 4 Mäusen, denen für Her-2/neu codierende mRNA (also der Sinnstrang) injiziert wurde, nach 9 Monaten tumorfrei.

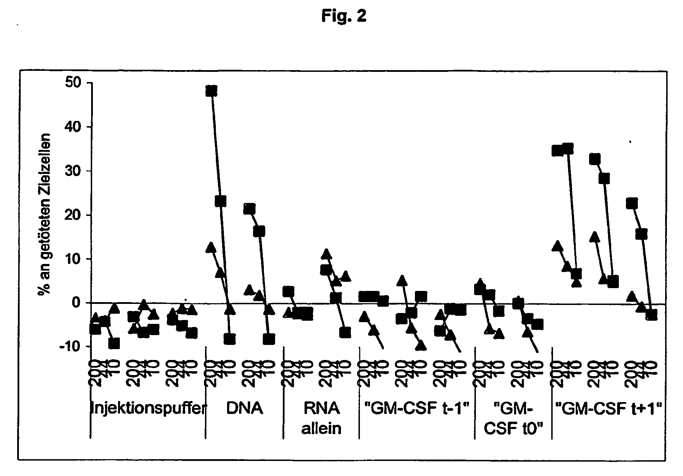

- Fig. 2: zeigt in einer graphischen Darstellung die Ergebnisse von Experimenten zur beta Galaktosidase (beta-Gal)-spezifischen CTL (cytotoxische T-Lymphocyten)-Aktivität durch die Immunisierung mit einer für beta-Gal codierenden mRNA unter dem Einfluß von GM-CSF. BALB/c-Mäuse wurden durch Injektion in die innere Auricula mit 25 µg für beta-Gal codierender mRNA immunisiert. Die Splenocyten wurden *in vitro* mit beta-Gal-Protein stimuliert, und die CTL-Aktivität wurde 6 Tage nach der *in vitro*-Stimulation unter Verwendung eines Standard-⁵¹Cr-Freisetzungstests bestimmt. Die Zielzellen waren P815 (H₂^{d})-Zellen, die mit dem synthetischen Peptid TPHPA-RIGL, das dem H₂^{d}-Epitop von beta-Gal entspricht, beladen (■) oder nicht beladen (▲) waren. Es wurden jeweils drei oder zwei Tiere pro Gruppe behandelt. Als Negativkontrolle dienten Tiere, denen i.d. in beide Auriculae nur Injektionspuffer injiziert wurden. Als Positivkontrolle ("DNA") dienten Tiere, denen in beide Auriculae i.d. 10 µg eines für beta-Gal codierenden Plasmids in PBS injiziert wurde. Die Testgruppen erhielten für beta-Gal codierende RNA allein bzw. in Kombination mit GM-CSF, der 24 h ("GM-CSF t-1"), 2 h vor der RNA.-Injektion ("GM-CSF t0") oder 24 h nach der RNA-Injektion ("GM-CFS t+1") in dieselbe Stelle (in die Auriculae) oder an einer anderen Stelle (s.c. auf dem Rücken) injiziert wurde. Es wurden jeweils drei verschiedene Effektor-/Zielzellen-Verhätlnisse (200, 44, 10) getestet.

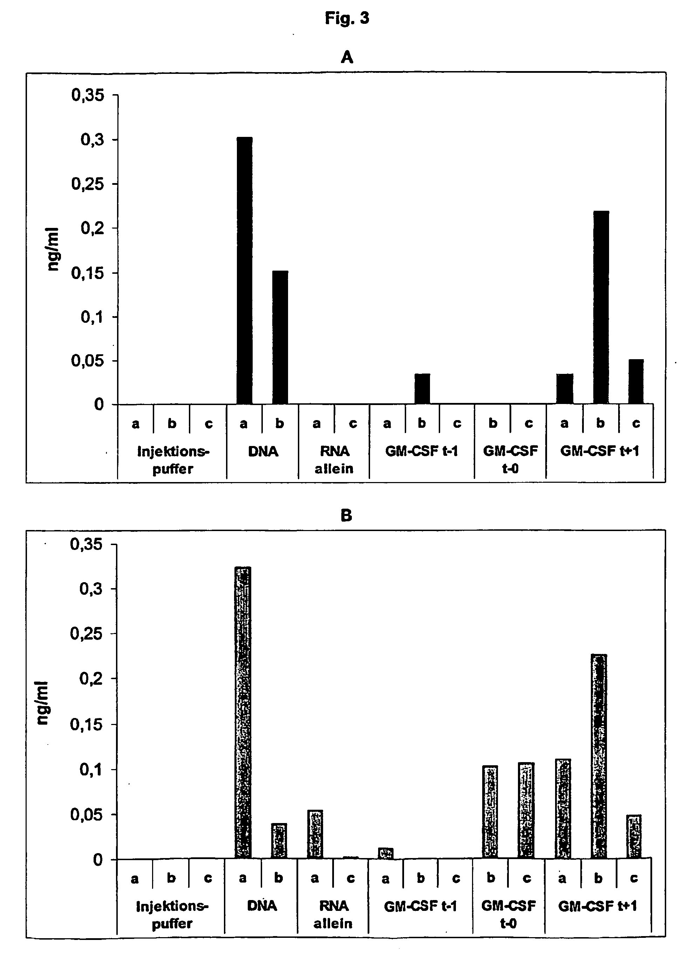

- Fig. 3: zeigt in weiteren graphischen Darstellungen die Ergebnisse von für IFN-gamma (A) bzw. IL-4 (B) spezifische ELISA-Standardtests, die die entsprechende Cytokin-Produktion von Splenocyten dokumentieren, die *in vitro* mit beta-Gal-Protein restimuliert wurden. BALB/c-Mäuse wurden, wie bereits vorstehend bei Fig. 2 angegeben, immunisiert. Die Splenocyten wurden *in vitro* mit beta-Gal-Protein stimuliert, die entsprechenden Kulturüberstände wurden gewonnen, und die IFN-gamma- oder IL-4-Konzentration wurde unter Verwendung eines ELISA-Standardtests bestimmt.

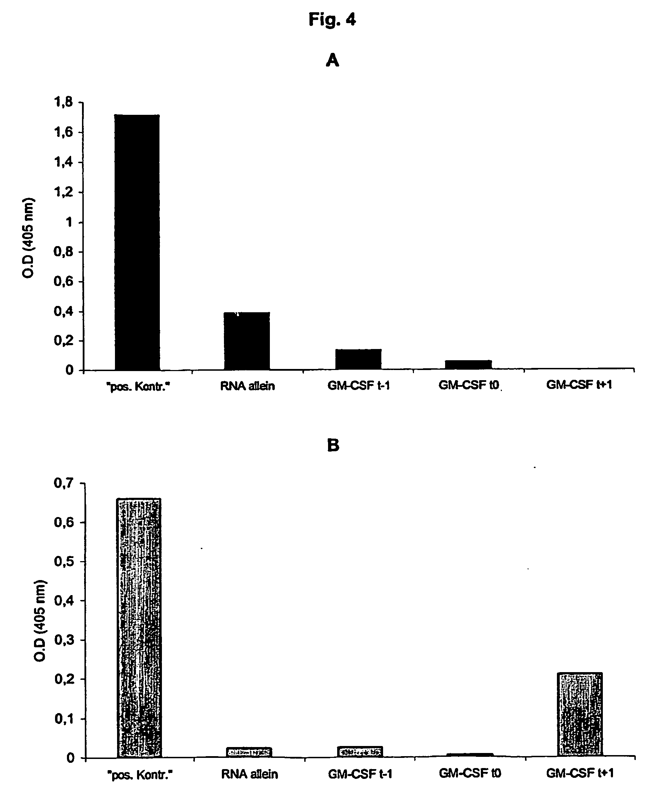

- Fig. 4: zeigt weitere graphische Darstellungen, welche die Antikörper-Antwort von erfindungsgemäß immunisierten Mäusen demonstriert. BALB/c-Mäuse wurden, wie in Fig. 2 angegeben, immunisiert. Zwei Wochen nach dem Boost wurde Blut abgenommen und daraus das Blutserum gewonnen. Beta-Gal-spezifische IgG1- (A) und IgG2a-Antikörper (B) wurden mit Hilfe eines ELISA-Test bestimmt. Dargestellt ist jeweils die Extinktion (OD) bei 405 nm auf der y-Achse, die aus der Umsetzung des Substrats ABTS beim ELISA-Test resultiert. Die gezeigten Extinktionen sind die Werte, von denen die entsprechenden Werte von mit Injektionspuffer behandelten Mäusen subtrahiert sind.

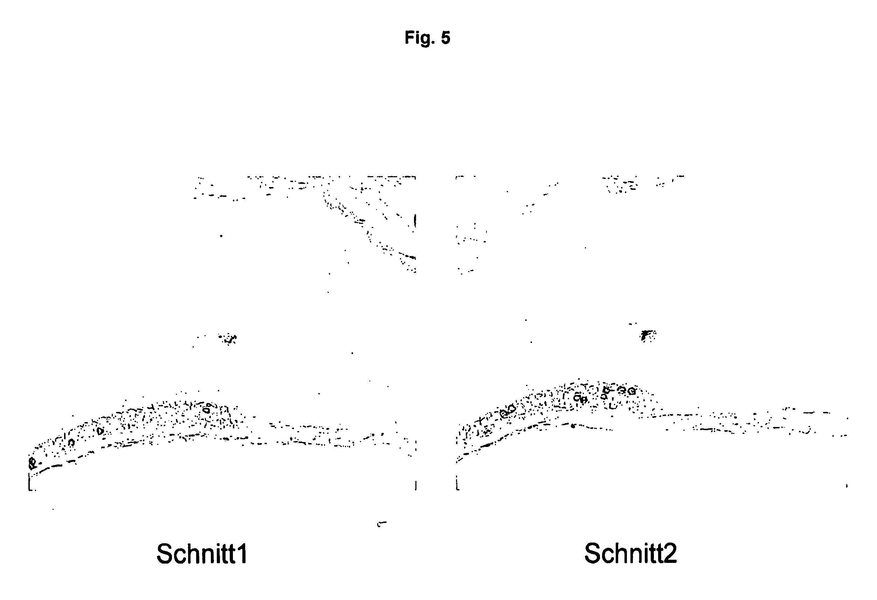

- Fig. 5: zeigt mit X-Gal gefärbte mikroskopische Schnitte der Auricula von Mäusen, denen für beta-Galaktosidase codierende mRNA in die Auricula i.d. injiziert wurde. 12 Stunden nach der Injektion von 25 *µ*g RNA in HEPES-NaCl-Injektionspuffer wurden die Ohren entfernt und X-Gal-gefärbte Schnitte angefertigt. Blaue Zellen zeigen eine beta-Galaktosidase-Aktivität an. Wie bei beiden Schnitten zu sehen ist, sind nur wenige blaue Zellen vorhanden.

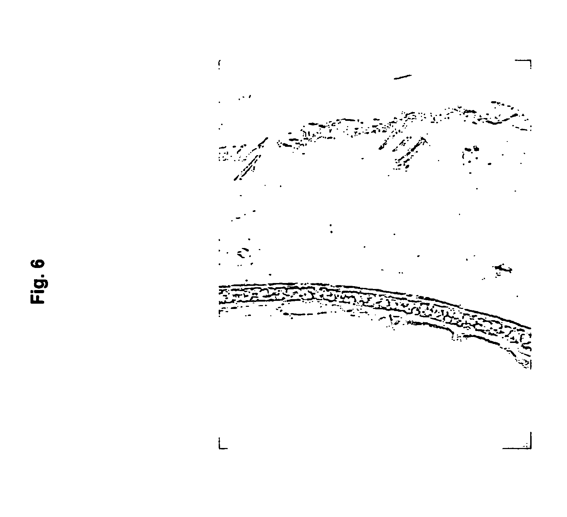

- Fig. 6: zeigt einen der Fig. 5 entsprechenden Schnitt durch eine Auricula einer Maus, der in die Auricula für beta-Galaktosidase codierende mRNA injiziert wurde, welche Protamin-stabilisiert war. Der mit X-Gal gefärbte mikroskopische Schnitt zeigt einige wenige blau gefärbte Zellen.

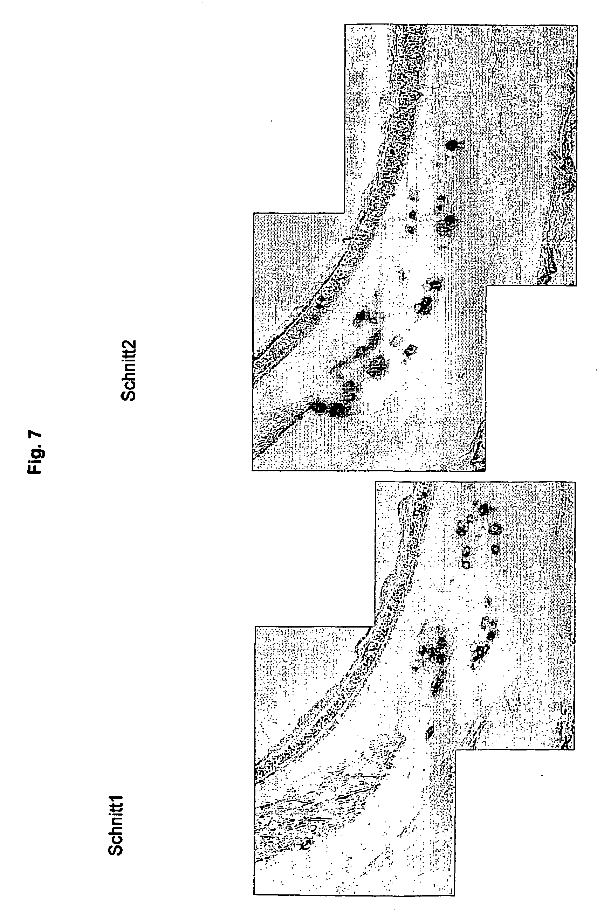

- Fig. 7: zeigt zwei weitere Schnitte durch die Auricula von Mäusen, wobei pro Schnitt zwei Bilder angefertigt wurden, um einen größeren Ausschnitt darzustellen. In diesem Fall wurde in die Auricula für beta-Galaktosidase codierende mRNA in einem Puffer injiziert, dem 10 U RNasin, ein enzymatischer RNase-Inhibitor aus Pankreas (erhältlich von Roche oder Promega) direkt vor der Injektion zugefügt wurde. Im Vergleich zu den Schnitten von Fig. 5 und Fig. 6 sind deutlich mehr blau gefärbte Bereiche von Zellen mit beta-Galaktosidase-Aktivität zu erkennen.
- Fig. 8: zeigt in einer schematischen Darstellung das Plasmid pT7TS, das für die *In vitro* - Transkription verwendet wurde. Erfindungsgemäße Konstrukte wurden in die BglII- und SpeI-Stellen, deren relative Lage zueinander angegeben ist, cloniert. Der schwarz ausgefüllte Bereich enthält die 5'-nicht translatierte Region des beta-Globingens aus *Xenopus laevis,* während der grau ausgefüllte Bereich eine entsprechende 3'-nicht translatierte Region des beta-Globingens aus *X*. *laevis* darstellt. Des Weiteren ist die relative Lage des T7-Promotors, die zur Sequenzierung verwendete PstI-Stelle, der Poly(A⁺)-Schwanz (A₃₀C₃₀) sowie mit einem Pfeil die Transkriptionsrichtung angegeben.

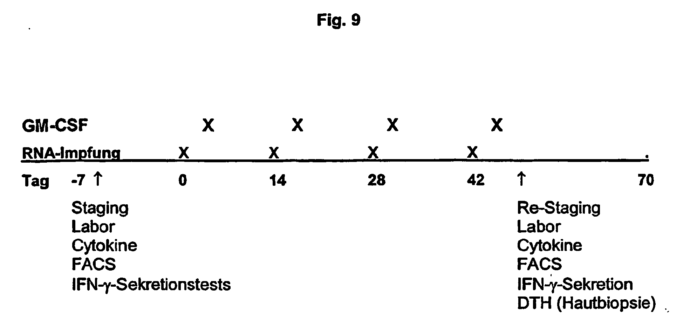

- Fig. 9: zeigt in einem beispielhaften Ablaufschema den Verlauf einer erfindungsgemäßen RNA-Impftherapie mit unterstützender Gabe von GM-CFS. Die für ein oder mehrere Tumorantigene (MUC1, Her-2/neu, Tilomerase, MAGE-1) codierende mRNA-Moleküle bzw. eine für ein Kontrollantigen (Influenza Matrix Protein (IMP), ein virales Antigen) codierende mRNA werden dem Patienten i.d. an den Tagen 0,14, 28 und 42 verabreicht. Zusätzlich wird dem Patienten einen Tag nach der RNA-Impfung GMCFS (Leucomax^{®} (100 µg/m²) von Novartis/Essex Pharma) s.c. injiziert. Bei stabilem Verlauf bzw. objektivem Tumoransprechen (komplette Remission (CR) bzw. partielle Remission (PR)) erhalten die Patienten die Vakzinierungen einmal im Monat s.c. Nach der vierten Injektion (Tag 49) wird das Ansprechen des Tumors radiologisch, laborchemisch bzw. sonographisch sowie die durch die Therapie induzierten immunologischen Phänomene evaluiert. Ab Tag 70 erfolgt die Fortführung der Immunisierungstherapie in 4-wöchentlichen Abständen. Am Tag 0, 14, 28, 42 und 49 werden Blutproben zur Bestimmung entsprechender Laborparameter, des Differenzialblutbilds (Diff-BB), FACS-Analyse und Cytokine entnommen. Das Restaging des Patienten erfolgt ab Tag 49 sowie ggf. alle weitere 4 bis 8 Wochen.

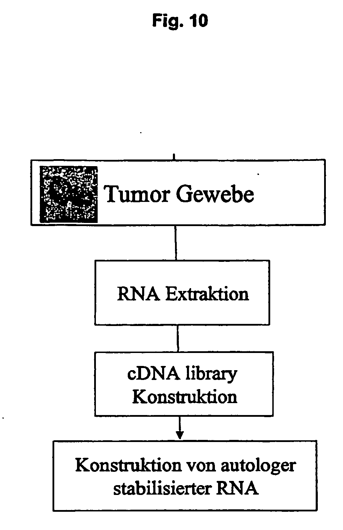

- Fig. 10: zeigt in einem Fließschema die Konstruktion von autologer, stabilisierter RNA gemäß dem Herstellungsverfahren der vorliegenden Erfindung. Zunächst wird Tumorgewebe, bspw. durch Biopsie, gewonnen. Daraus wird die Gesamt-RNA extrahiert. Anhand der aus der RNA-Extraktion gewonnenen Poly(A⁺)-RNA wird eine cDNA-Bibliothek konstruiert. Davon ausgehend wird nach Herstellung einer entsprechenden DNA-Matrize die autologe, stabilisierte RNA mittels *In vitro*-Transkription gewonnen.
- Fig. 11: zeigt in einem Reaktionsschema die Schritte zur Herstellung einer cDNA-Bibliothek, ausgehend von Poly(A⁺)-RNA beispielhaft für das SMART PCR cDNA Synthesis Kit der Fa. Clontech Inc.

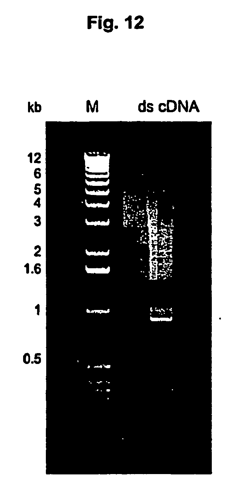

- Fig. 12: zeigt eine fotographische Aufnahme eines Agarosegels, das die typische Größenfraktionierung einer cDNA Bibliothek, erstellt aus humanem Placenta-Gewebe, zeigt. In der Spur M ist ein Längenmarker mit Fragmenten der links angegebenen Länge aufgetragen. Die Spur "DS cDNA" enthält die cDNA Bibliothek. Diejenigen Fragmente, die der erwarteten Größenfraktion entsprechen (etwa 200 BP bis 4000 BP) werden für die In *vitro* Transkription verwendet.

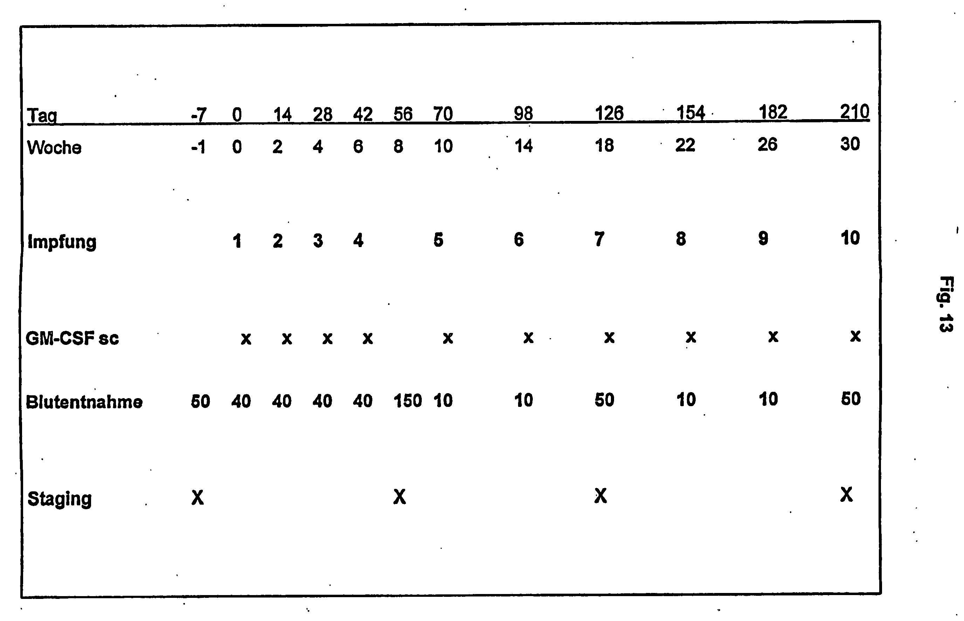

- Fig. 13: zeigt einen beispielhaften Behandlungsplan für die erfindungsgemäße Tumor-Therapie durch Injektion einer Tumor-mRNA-Bibliothek, hier in Kombination mit GM-CSF, für Patienten mit malignem Melanom. Hierfür wird aus patienreneigenem Tumorgewebe hergestellte, autologe, stabilisierte RNA verwendet Diese amplifizierte autologe Tumor-RNA wird dem Patienten i.d. an den Tagen 0, 14, 28 und 42 verabreicht. Zusätzlich wird dem Patienten einen Tag nach der RNA-Injektion GM-CFS (Leucomax^{®} 100 µg/m² Novartis/Essex Pharma) s.c. injiziert. Zwei Wochen nach der vierten Injektion (Tag 56) wird das Ansprechen des Tumors durch eine Staging-Untersuchung (u.a. Sonographie, Thorax-Röntgen, CT usw.) sowie durch die Auswertung der durch die Therapie induzierten immunologischen Parameter evaluiert. Bei stabilem Krankheitsverlauf bzw. objektivem Tumoransprechen (CR bzw. PR) erhält der Patient alle vier Wochen jeweils eine weitere Vakzinierung. Weitere Restaging-Untersuchungen werden am Tag 126 und danach in 12-wöchigem Abstand durchgeführt.

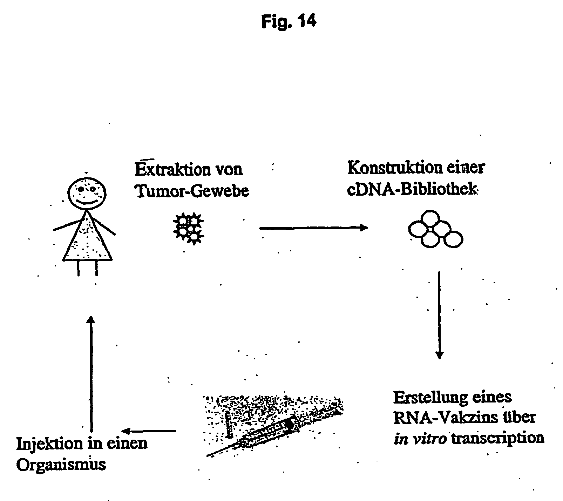

- Fig. 14: zeigt noch einmal schematisch den generellen Ablauf einer Therapie mit der erfindungsgemäßen pharmazeutischen Zusammensetzung mit autologer, amplifizerter Tumor-RNA, d.h. die in der pharmazeutischen Zusammensetzung enthaltene RNA repräsentiert eine cDNA-Bibliothek des Tumorgewebes. Es wird zunächst eine Probe des Tumorgewebes, bspw. über eine Biopsie, gewonnen. Aus dem Gewebe wird die Gesamt-, dann die Poly(A+)-RNA durch entsprechende Extraktionen hergestellt. Ausgehend von der Poly(A+)-RNA wird eine cDNA-Bibliothek konstruiert, die in einen zur nachfolgenden *In vitro*-Transkription geeigneten Vektor kloniert wird. Durch *In vitro*-Transkription wird dann ein RNA-Vakzin gewonnen, das dem Patienten, dem das Tumorgewebe entnommen wurde, zur Bekämpfung des Tumors injiziert wird.

Die nachfolgenden Ausführungsbeispiele erläutern die vorliegende Erfindung näher, ohne sie einzuschränken.

### BEISPIELE

### Beispiel 1: Tumorvakzinierung mit RNA im Tiermodell

### Materialien und Methoden

Gekappte mRNA, codierend für eine verkürzte Version des Her-2/neu-Proteins der Ratte ("ECD-TM-neu-Ratte", enthaltend die extrazelluläre Domäne und die Transmembranregion, nicht jedoch die cytoplasmatische Region) wurde unter der Verwendung von "SP6 mMessagemMachine" (Ambion) mit Hilfe eines Plasmids hergestellt, das im wesentlichen dem in Fig. 8 gezeigten Aufbau entsprach, jedoch statt des T7-Promotors einen SP6-Promotor enthielt, und in welchem das ECD-TM-neu-Ratte-Konstrukt hinter den SP6-RNA-Polymerase-Promotor eingefügt war. Die hergestellte mRNA wurde in Injektionspuffer (150 mM NaCl, 10 mM HEPES) bei einer Konzentration von 0,8 mg/ml gelöst und mit Protamin-Sulfat (Sigma) (1 mg Protamin pro 1 mg RNA) gemischt. 50 *µ*l dieser Lösung wurden in die Auriculae (je 25 *µ*l pro Ohr) der Mäuse injiziert. Es erfolgten acht Injektionen, jeweils eine im Alter von 6, 8, 13, 15, 20, 22, 27 und 29 Wochen. Als Kontrollen dienten Mäuse, denen entsprechende Injektionen mit Injektionspuffer, mit für ECD-TM-neu-Ratte codierender Plasmid-DNA oder mit einer der erfindungsgemäßen mRNA entsprechenden Antisense-mRNA verabreicht wurden.

### Ergebnisse

Weibliche BalB-neu T-Mäuse (BalB/c-Mäuse, die das Onkogen Her2/neu der Ratte exprimieren; vgL Rovero et al. (2000) J. Immunol. 165(9):5133-5142), die spontan Mammakarzinome entwickeln, wurden mit für eine verkürzte Version des Her-2/neu-Proteins ("ECD-TM-neu-Ratte", enthaltend die extrazelluläre Domäne und die Transmembranregion, nicht jedoch die cytoplasmatische Region) codierender RNA immunisiert. Als Negativkontrolle dienten vier mit Injektionspuffer behandelte Mäuse. Einer weiteren Gruppe von drei Mäusen wurde für das verkürzte Her-2/neu codierende DNA injiziert. Vier Mäuse erhielten die erfindungsgemäß für das Tumorantigen Her-2/neu (verkürzte Version ECD-TM, siehe oben) codierende mRNA. Als weitere Kontrollgruppe dienten vier Mäuse, denen die entsprechende Antisense-RNA injiziert wurde. Wie in der Fig. 1 gezeigt, wurde bei den Tieren der unbehandelten Kontrollgruppe nach 26 Wochen eine Tumormultiplizität von durchschnittlich 10 beobachtet, wobei alle Tiere im Alter von etwa 20 Wochen tastbare Brusttumore aufwiesen. Im Gegensatz dazu ist bei der Immunisierung mit der für ECD-TM-neu-Ratte codierenden mRNA eine deutliche Verlangsamung der Karzinomentstehung zu beobachten, insbesondere wird erst im Alter von 30 Wochen eine Tumormultiplizität von 10 erreicht. Des Weiteren ist auch die Tumorgröße vermindert (nicht gezeigt). Von den 4 mit der erfindungsgemäßen mRNA behandelten Mäusen war eine nach 9 Monaten immer noch tumorfrei. Diejenige Gruppe von Mäusen, denen die Antisense-mRNA injiziert worden war, zeigten alle im Alter von 6 Monaten Tumore. Die Vergleichsgruppe der Mäuse, denen für die verkürzte Version von Her-2/neu codierende Plamid-DNA injiziert wurde, zeigten ebenfalls eine im Vergleich zur unbehandelten Kontrollgruppe verlangsamte Karzinomentstehung (vgL auch bezüglich entsprechender Plasmid-DNA-Experimente bei intramuskulärer Injektion: Di Carlo et aL (2001) Clin. Cancer Res. 7(3. Ergänzungsband): 830s-837s), wobei jedoch die Karzinomentstehung bis zur 27. Lebenswoche nicht so stark verlangsamt war wie bei der Immunisierung mit erfindungsgemäßer, für die verkürzte Version von Her2/neu codierender mRNA. Darüber hinaus sind bei der Immunisierung mit DNA die vorstehend genannten Nachteile, insbesondere die Gefahr der Integration der DNA in das Genom, die Entstehung von anti-DNA-Antikörpern usw. zu berücksichtigen.

### Beispiel 2: Einfluss von GM-CFS auf die RNA-Vakzinierung

### Materialien und Methoden

### Mäuse

6-10 Wochen alte BALB-c AnNCrIBR (H-2^{d}) Mäuse (weiblich) wurden von Charles River (Sulzfeld, Deutschland) bezogen.

### Plasmide und Herstellung von RNA

Der für beta-Galaktosidase codierende ORF (LacZ), flankiert von 5'- und 3'-nicht-translatierten Sequenzen aus dem beta-Globingen von *X. Laevis,* wurde in das Plasmid pT7TS (P.A. Creek, Austin, TX, USA), um das Plasmid pT7TS-kozak-5' beta gl-lacZ-3' beta gl-A30C30 herzustellen (vgL Hoerr et aL (2000) Eur. J. ImmonoL 30:1-7). Der generelle Aufbau des Plasmids pT7TS mit den flankierenden 5'- und 3'- nicht translatierten Sequenzen aus dem beta-Globingen von *X. Laevis* ist in Fig. 8 schematisch dargestellt.

Das derart hergestellte Plasmid wurde mit Pst I linearisiert und *in vitro* unter Verwendung des m-MessagemMachine T7 Kit (Ambion, Austin, TX USA) transkripiert. Die so hergestellte RNA wurde mittels LiCl-Präzipitation, Phenol/Choroform-Extraktion und Ammoniumacetat-Fällung gereinigt. Die gereinigte RNA wurde schließlich in einer Konzentration von 0,5 mg/ml in Injektionspuffer (150 mM NaCl, 10 mM HEPES) resuspendiert.

### Medien und Zellkultur

P815 und P13.1-Zellen wurden in RPMI 1640 (Bio-Whittaker, Verviers, Belgien), ergänzt mit 10% Hitze-inaktiviertem fötalem Kälberserum (FCS) (PAN systems, Deutschland), 2 mM L-Glutamin, 100 U/ml Penicillin und 100 mg/ml Streptomycin, kultiviert.

CTL-Kulturen wurden in RPMI 1640-Medium, ergänzt mit 10 % FCS, 2 mM L-Glutamin, 100 U/ml Penicillin, 100 mg/ml Streptomycin, 0,05 µM beta-Mercaptoethanol, 50 mg/ml Gentamycin, MEM-Non Essential Amino Assids (100 x) und 1 mM Natriumpyruvat, gehalten. CTL wurden für eine Woche mit 1 mg/ml beta-Galaktosidaseprotein (Sigma, Taufhrchen, Deutschland) restimuliert. Am Tag 4 wurden 4 ml Kulturüberstand vorsichtig abpipettiert und durch frisches Medium, enthaltend 10 U/ml rIL-2 (Endkonzentration) ersetzt.

### Immunisierung

3 BALB/c-Mäuse pro Gruppe wurden mit 20 mg Pentobarbital i.p. pro Maus betäubt Den Mäusen wurde dann i.d. in beide Auriculae 25 mg für beta-Galaktosidase (beta-Gal) codierende mRNA Injektionspuffer (150 mM NaCl, 10 mM HEPES) injiziert In einigen Fällen wurde zusätzlich Granomycyten-Makrophagen-Kolonie-Stimulisierungsfaktor (GM-CFS) 24 h oder 2 h vor bzw. 24 h nach der RNA-Injektion in dieselbe Stelle oder in eine davon entfernte Injektionsstelle (in die Auricula oder s.c. in den Rücken) injiziert Als Positivkontrolle wurde Tieren jeweils 10 mg eines für beta-Gal codierenden DNA-Plasmids in PBS i.d. in beide Auriculae injiziert. Eine Gruppe von Tieren, denen in beide Auriculae nur Injektionspuffer i.d. appliziert wurde, diente als Negativkontrolle. Zwei Wochen nach der Erstinjektion wurde eine Boost-Injektion in jeweils der gleichen Weise wie bei der ersten Injektion durchgeführt. Zwei Wochen nach der Boost-Injektion wurde Blut genommen, die Mäuse wurden getötet und die Milz entfernt

### ⁵¹Cr-Freisetzungstest

Aus der Milz erhaltene Splenocyten wurden *in vitro* mit beta-Gal-Protein- stimuliert und die CIL-Aktivität wurde nach 6 Tagen unter Verwendung eines 6stündigen ⁵¹-Cr-Standardtersts, wie in Rammensee et al. (1989) Immimogenetics 30: 296 - 302, beschrieben, bestimmt. Kurz zusammengefasst wurden Zielzellen mit ⁵¹Cr markiert und mit dem Peptid TPHPARIGL für 20 min bei Raumtemperatur beladen. Nach der Co-Inkubation von Effektor- und Zielzellen (bei jeweils drei verschiedenen Verhältnissen von Effktor:Zielzellen: 200, 44 und 10) in runden Platten mit 96 Vertiefungen für 6 h wurden 50 ml von 200 ml Kulturüberstand in eine Luma-Szintillationsplatte (Packard) mit 96 Verdefungen pipettiert, und nach dem Trocknen wurde die Radioaktivität mit einem Szintillationszähler (1405 Microbeta Plus) gemessen. Die prozentuale spezifische Freisetzung wurde aus der Menge des in das Medium freigesetzten ⁵¹Cr (A) minus der spontanen Freisetzung (B) geteilt durch die Gesamtfreisetzung (C) (unter Verwendung von Triton X-100) minus der spontanen Freisetzung (B) bestimmt Prozent spezifische Lyse = 100 (A-B)/(C-B).

### Cytokin-ELISA

Nach 4 Tagen der Restimulierung mit beta-Gal-Protein wurde der Überstand der Splenocytenkultur abpipettiert und bei - 50°C bis zur Verwendung gelagert. Auf MaxiSorb-Platten (Nalge Nunc International, Nalge, Dänemark) wurden über Nacht bei 4°C 100 ml Anti-Maus-Anti-IFN-gamma- oder -IL-4-Fängerantikörper (Becton Dickenson, Heidelberg, Deutschland bei einer Konzentration von 1 mg/ml in Beschichtungspuffer (0,02 % NaN₃, 15 mM Na₂CO₃, 15 mM NaHCO₃, BH 9,6) auspipettiert. Nach dreimaligem Waschen mit Waschpuffer (0,05 % Tween 20 in PBS) wurden die Platten mit 200 ml Blockingpuffer (0,05 % Twen 20,1 % BSA in PBS) für 2 h bei 37° C abgesättigt. Nach dreimaligem Waschen mit Waschpuffer wurden 100 ml der Zellkulturüberstände für 5 h bei 37° C inkubiert. Die Platten wurden dann viermal mit Waschpuffer gewaschen, und es wurden 100 ml biotinylierte Anti-Maus-Anti-IFN-gamma- oder -IL-4-Detektionsantikörper (Becton Dickenson, Heidelberg, Deutschland) pro Vertiefung bei einer Konzentration von 0,5 mg/ml in Blockierungspuffer pipettiert und für 1 h bei Raumtemperatur inkubiert. Nach dreimaligem Waschen mit Waschpuffer wurden in jede Vertiefung 100 ml einer 1/1000-Verdünnung Spreptavidin-HRP (BD Biosciences, Heidelberg, Deutschland) gegeben. Nach 30 min bei Raumtemperatur wurden die Platten dreimal mit Waschpuffer und zweimal mit bidestilliertern Wasser gewaschen. Danach wurden in jede Vertiefung 100 ml des ABTS-Substrats zugegeben. Nach 15 - 30 min bei Raumtemperatur wurde die Extinktion bei 405 um mit einem Sunrise-ELISA-Lesegerät (Tecan, Crailsheim, Deutschland) gemessen.

### Antikörper-ELISA

Zwei Wochen nach der Boost-Injektion wurde den Mäusen Blut über die Orbitalvene abgenommen und Blutserum hergestellt. Auf Maxisorb-Platten (Nalge Nunc International, Nalge, Dänemark) wurden für 2 h bei 37° C 100 ml beta-Gal-Protein in einer Konzentration von 100 mg/ml in Beschichtungspuffer (0,05 M Tris-HCl, 0,15 M NaCl, 5 mM CaCl₂, pH 7,5) auspipettiert. Dann wurden die Platten dreimal mit 200 ml Waschpuffer (0,05 M Tris-HCl, 0,15 M NaCl, 0,01 M EDTA, 0,1 % Tween 20,1 % BSA, pH 7,4) gewaschen und mit 200 ml Waschpuffer über Nacht bei 4° C mit Protein abgesättigt. Die Platten wurden dreimal mit Waschpuffer gewaschen, und es wurden Blut-Seren in einer Verdünnung von 1/10, 1/30 bzw. 1/90 in Waschpuffer zugegeben. Nach 1 h bei 37° C wurden die Platten dreimal mit Waschpuffer gewaschen, und es wurden 100 ml von 1/1000-Veidünnungen von Ziege-Ami-Maus-IgG1- oder -IgG2a-Antikörpern (Caltag, Burlington, CA, USA) zugegeben. Nach 1 h bei Raumtemperatur wurden die Vertiefungen dreimal mit Waschpuffer gewaschen, und es wurden 100 ml ABTS-Substrat pro Vertiefung zugefügt Nach 15 - 30 min bei Raumtemperatur wurde die Extinktion bei 405 nm mit einem Sunrise-ELISA-Lesegerät (Tecan, Crailsheim, Deutschland) gemessen.

### Ergebnisse und Diskussion

Es wurde bestätigt, daß die direkte Injektion von RNA, die für beta-Galaktosidase codiert, in die Auricula von Mäusen eine Anti-beta-Galaktosidase-Immunantwort, im wesentlichen vom Th2-Typ, auslöst: Es wird eine Produktion von Anti-beta-Galaktosidase-Immunglobulinen vom IG1-Typ (Fig. 3A) und eine Sekretion von IL-4 (Fig. 2 B) bei mit beta-Galaktosidasestimulierten Splenocyten von Mäusen festgestellt, denen die für beta-Galaktosidase codierende RNA injiziert wurde. Um die Effizienz der RNA-Vakzine zu verstärken, wurde zusätzlich das Cytokin GM-CFS verabreicht. Dieses Cytokin verstärkt die Effizienz bei einigen DNA-Vakzinen. Darüber hinaus wurde festgestellt, daß der Zeitpunkt der GM-CFS-Injektion im Vergleich zur DNA-Injektion den Typus der Immunantwort beeinflußt (Kusakabe (2000) J. Immunol. 164:3102-3111). Erfindungsgemäß wurde festgestellt, daß GM-CFS die durch eine RNA-Vakzinierung hervorgerufene Immunantwort verstärken kann. Die Injektion von GM-CFS einen Tag vor der Injektion von RNA zeigt kaum einen Einfluß auf die Stärke oder den Typus der Immunantwort. Im Gegensatz dazu verstärkt die Injektion von GM-CFS 2 Stunden vor der Injektion der RNA die Immunantwort (vgl. die IL-4-Freisetzung in Fig. 2B bei den 2 Mäusen, denen GM-CFS beim Zeitpunkt T = 0 injiziert wurde), beeinflußt jedoch nicht die Th2-Polarität. Wird GM-CFS dagegen einen Tag nach der RNA-Vakzine in dieselbe Stelle oder in eine davon entfernte Stelle (nicht gezeigt) injiziert, wird die Immunantwort nicht nur insgesamt verstärkt (vgl. die Antikörper-Antwort gemäß Fig. 3), sondern die Immunantwort wird zum Th1-Typ polarisiert (vgl. die IFN-gamma-Produktion durch beta-Gal-Protein-stimulierte Splenocyten gemäß Fig. 2A, die Produktion von IgG2a-Antikörpem gegen beta-Gal gemäß Fig. 3B und die Produktion aktivierter CTL gemäß Fig. 1). Die Injektion von GM-CFS einige Minuten oder einige Stunden nach der RNA-Injektion sollte die gleiche Auswirkung (Verstärkung und Polarisierung) der Immunantwort ergeben.

### Beispiel 3: Auswirkung von RNase-Inhibitor auf die mRNA-Expression in vivo

Es wurde nackte oder protamin-assoziierte bzw. -komplexierte mRNA, die für beta-Galaktosidase codiert (hergestellt wie in Beispiel 2 angegeben) in die Auricula von Mäusen in einer Menge von 25 mg RNA in Injektionspuffer (150 mM NaCl, 10 mM HEPES) injiziert. Weiteren Mäusen wurde die für beta-Galaktosidase codierende mRNA zusammen mit 10 U des RNase-Inhibitors RNasin (ein aus Pankreas extrahierter enzymatischer RNase-Inhbitor, erhaltlich von Roche oder Promega) injiziert. Der RNase-Inhibitor wurde unmittelbar mit der RNA-Lösung vor der Injektion vermischt. Nach 12 Stunden wurden jeweils den Mäusen die Ohren abgenommen. Mikroskopische Dünnschnitte der Auriculae wurden hergestellt und mit X-Gal gefärbt. Die Injektion nackter oder protamin-assoziierter mRNA führt zu einer detektierbaren beta-Galaktosidase-Aktivität in einigen wenigen Zellen bei den entsprechenden Dünnschnitten (blaue Zellen in Fig. 5 und 6). Somit haben hier einige Zellen die exogene RNA aufgenommen und in das Protein translatiert. Wenn die für beta-Galaktosidase codierende mRNA mit dem RNase-Inhibitor RNasin geschützt vorlag, wurden sehr viel mehr blaue Zellen als im Fall der nackten oder protamin-assoziieiten RNA beobachtet (Fig. 7). Da RNasin RNasen inhibiert, wird die Halbwertszeit der injizierten mRNA-Moleküle *in vivo,* dort wo die Umgebung (interstitielles Gewebe) mit RNasen kontaminiert ist, verlängert. Die derartige Stabilisierung der RNA führt zu einer verstärkten Aufnahme durch die umgebenden Zellen und dadurch zu einer verstärkten Expression des von der exogenen RNA codierten Proteins. Diese Phänomen kann daher auch für eine verstärkte Immunantwort gegen ein von der injizierten mRNA codierten Antigen genutzt werden.

### Beispiel 4: RNA-Vakzinierung bei Patienten mit malignen Erkrankungen

### Einleitung

Cytotoxische T-Lymphocyten (CTL) erkennen Antigene als kurze Peptide (8-9 Aminosäuren), die an MHC Klasse I Glykoproteine gebunden an der Zelloberfläche exprimiert werden (1). Diese Peptide sind Fragmente intrazellulärer Eiweißmoleküle. Es gibt jedoch Hinweise, daß auch exogen durch Makropinocytose oder Phagocytose aufgenommene Antigene zur CD8⁺ T-Zell-vermittelten Immunantwort führen können. Die Proteine werden in Proteosomen gespalten, die hierbei entstehenden Peptide werden aus dem Cytosol in das Lumen des endoplasmatischen Retikulums transportiert und an MHC Klasse I Moleküle gebunden.

Die so prozessierten Proteine werden als Peptid/MHC-Klasse I-Komplex an die Zelloberfläche transportiert und den CIL präsentiert. Dieser Vorgang findet in jeder Zelle statt und ermöglicht so dem Immunsystem ein genaues Überwachen jeder einzelnen Zelle auf das Vorhandensein körperfremder bzw. veränderter oder embryonaler Proteine, unabhängig davon ob sie von intrazellulären pathogenen Keimen, Onkogenen oder dysregulierten Genen stammen. Dadurch sind cytotoxische Lymphocyten in der Lage, infizierte bzw. neoplastische Zellen zu erkennen und zu lysieren (2, 3).

In den letzten Jahren ist es gelungen, verschiedene tumor-assozierte Antigene (TAA) und Peptide, die von CIL erkannt werden und dadurch zur Lyse von Tumorzellen führen, zu isolieren (21-27). Diese TAA sind in der Lage, T-Zellen zu stimulieren und antigenspezifische CTL zu induzieren, wenn sie als Komplex aus HLA-Molekül und Peptid auf AntigenprSsentierenden Zellen (APC) exprimiert werden.

In zahlreichen Untersuchungen, die vor allem bei Patienten mit malignem Melanom durchgeführt wurden, konnte gezeigt werden, dass maligne Zellen beim Fortschreiten der Tumorerkrankung die Expression von TAA verlieren. Ähnliches wird auch bei Vakzinierungen mit einzelnen Tumorantigenen beobachtet. Unter Vakzinierungstherapien kann es auch zur Selektion von Tumorzellen kommen, was ein Entkommen vom Immunsystem und eine Progression der Erkrankung trotz Therapie ermöglicht. Die Anwendung von mehreren verschiedenen Tumorantigenen wie im erfindungsgemäßen Behandlungsplan des vorliegenden Beispiels vorgesehen, soll eine Selektion von Tumorzellen sowie ein Entkommen der malignen Zellen vom Immunsystem durch Antigenverlust verhindern.

Vor kurzem wurde eine Methode, mit der DC mit RNA aus einem Plasmid, das ein Tumorantigen kodiert, transfiziert werden können, entwickelt (Nair et aL, 1998, Nair et aL, 2000). Die Transfektion von DC mit RNA für CEA oder Telomerase führte zur Induktion von Antigen-spezifischen CTL. Dieses Verfahren ermöglicht es, CTL sowie T-Helfer Zellen gegen mehrere Epiope auf verschiedenen HLA-Molekülen aus einem Tumorantigen zu induzieren. Ein weiterer Vorteil dieser Strategie ist die Tatsache, dass sie weder die Charakterisierung der verwendeten Tumorantigene bzw. Epitope noch die Definition des HLA-Haplotyps des Patienten voraussetzt. Durch eine derartige polyvalente Vakzine könnte die Wahrscheinlichkeit für das Auftreten von sogenannten klonalen "tumor escape"- Phänomenen deutlich gesenkt werden. Darüber hinaus könnten durch diesen Ansatz T-Zell-vermittelte Immunantworten gegen auf natürlichem Wege prozessierte und präsentierte Antigene mit eventuell höherer Immundominanz induziert werden. Durch zusätzliche Beteiligung von MHC-Klasse II restringierten Epitopen könnte die induzierte tumorspezifische Immunantwort verstärkt und länger aufrecht erhalten werden.

Es wird beispielhaft ein erfindungsgemäßes Behandlungsschema zur Tumor-Vakzinierung von Patienten mit fortgeschrittenen malignen Erkrankungen (Mamma-, Ovarial-, kolorektale, Pankreas- und Nierenzellkarzinome) bereitgestellt. Hierbei wird RNA, die aus Plasmiden, die für MUC1, Her-2/neu, Telomerase und MAGE-1 Tumorantigene sowie Influenza-Matrix-Protein (IMP) (positive Kontrolle) codieren, hergestellt wurde, Patienten mit oben erwähnten malignen Erkrankungen intra dermal verabreicht. Dadurch wird eine CTL-Induktion *in vivo* ermöglicht, um so die Progression der Erkrankung zu verhindern bzw. deren Rückbildung zu bewirken. Die genannten Tumorantigene werden auf den malignen Zellen von Mamma-, Ovarial-, kolorektalen, Pankreas- und Nierenzellkarzinomen exprimiert.

Gemäß dem Bahandlungsplan (vgL die diesbezüglichen nachstehenden Ausführungen sowie Fig. 9) werden die im Labor hergestellten RNA-Spezies, die für CEA, MUC1, Her-2/neu, Telomerase, Mage-1 und IMP kodieren, den Patienten i.d., zunächst 4 x an den Tagen 0,14, 28 und 42, verabreicht. Zusätzlich wird den Patienten jeweils einen Tag nach der RNA-Impfung GM-CSF (Leucomax^{®}, 100 *µ*g/m², Novartis/Essex Pharma) s.c. verabreicht.

Bei der erfindungsgemäßen Behandlung handelt es sich um einen Immunisierungsansatz, der nur minimale Eingriffe beim Patienten (Injektion) erfordert. Die Therapie erfolgt ambulant und ist für viele Tumorpatienten geeignet ohne die Einschränkung auf bestimmte HLA-Typen oder definierte T-Zellepitope. Des Weiteren, können durch diese Therapie polyklonale GD4⁺-T-Helfer als auch CD8⁺-CTL induziert werden.

### Behandlungsplan

Die RNAs für mehrere Tumorantigene (MUC1, Her-2/neu, Telomerase, MAGE-1) und für ein Kontrollantigen, dem Influenzamatrixprotein (IMP, virales Antigen), werden dem Patienten i.d. an den Tagen 0,14, 28 und 42 verabreicht. Zusätzlich erhalten die Patienten jeweils einen Tag nach der RNA-Impfung GM-CSF (Leucomax^{®} (100 µg/m²) Novartis/Essex Pharma) s.c.. Bei stabilem Krankheitsverlauf bzw. einem objektiven Tumoransprechen (komplette Remission (CR) bzw. partielle Remission (PR)) erhalten die Patienten ggf. die Vakzinierungen einmal im Monat s.c. Nach der vierten Injektion (Tag 49) wird das Ansprechen des Tumors radiologisch, laborchemisch und/oder sonographisch sowie die durch die Therapie induzierten immunologischen Phänomene evaluiert.

Ab Tag 70 erfolgt eine Fortführung der Immunisierungstherapie in 4-wöchtentlichen Abständen.

An den Tagen 0, 14, 28, 42 und 49 werden jeweils Blutproben für Labor, Diff-BB, FACS-Analyse und Cytokine entnommen (insgesamt 50 ml). Das Restaging der Patienten erfolgt ab Tag 49 sowie ggf. alle weitere 4 bis 8 Wochen.

Der Behandlungsplan ist in der Fig. 9 schematisch dargestellt.
- Labor:: Gerinnung, Elektrolyte, LDH, β2-M, CK, Leberenzyme, Bilirubin, Kreatinin, Harnsäure, Gesamteiweiß, Gerinnung, CRP, Tumormarker (Ca 12-5, Ca 15-3, CEA, Ca 19-9):15 ml Blut.
- Diff-BB:: Differentialblutbild mit Ausstrich (5 ml EDTA-Blut).
- Cytokine:: 10 ml Serum.
- FACS:: 10 ml Heparin-Blut.
- ELIspot:: 20 ml Heparin-Blut.
- Multitest:: Analyse der DTH Reaktion.
- DTH:: (engl. "delayed type hypersensitivity", verspätete T-Zell-vermittelte Reaktion) Analyse der Reaktion auf intra dermal verabreichte RNA. Zusätzlich soll eine Hautbiopsie bei postiver DTH-Reaktion erfolgen (hierfür ist keine Lokalanästhesie erforderlich).

### Herstellung von RNA aus Plasmiden

Für die Produktion einer Vakzine auf mRNA-Basis werden nur chemisch synthetisierte und aus Bakterien aufgereinigte Vorstufen benötigt. Dies wird vorzugsweise in einer besonders ausgerüsteten RNA-Produktions-Einheit bewerkstelligt. Diese befindet sich in einem abgeschlossenen Raum, der als RNase-freie Zone deklariert ist, d.h. Arbeiten mit RNase (z.B. die Aufreinigung von Plasmiden) dürfen nicht ausgeführt werden. Ebenso wird die Kontamination mit natürlich vorkommenden RNasen stetig überprüft. Dieser Raum ist mit Neugeräten (4°C- und -20°C-Kühlschränke, Heizblock, Sterilbank, Zentrifuge, Pipetten) ausgestattet, welche noch nie in Benutzung für biologische oder klinische Arbeiten waren. Diese RNA-Produktionseinheit wird ausschließlich für die enzymatische Produktion (In *vitro* -Transkription) von mRNA genutzt (ohne bakterielle, virale oder Zellkultur-Arbeiten). Das Endprodukt umfasst eine sterile RNA-Lösung in HEPES/NaCl-Puffer. Qualitätsanalysen werden auf einem Formaldehyd-Agarosegel durchgeführt. Zusätzlich wird die RNA-Konzentration sowie der Anteil an Proteinen photometrisch bestimmt (OD₃₂₀ < 0,1; Verhältnis von OD₂₆₀/OD₂₈₀ > 1,8 bei reiner RNA). Eine mögliche Kontamination durch LPS wird im LAL-Test analysiert. Alle RNA-Proben werden vor dem Verabreichen steril filtriert.

### Plasmid-Konstrukte

Die ausgewählten Gene (CEA, Mucin1, Her-2/neu, Telomerase, Mage-A1 and Influenza Matrix) werden über PCR durch den Einsatz eines thermostabilen High-Peformance-Enzyms (pfu, Stratagene) amplifiziert. Die Gene stammen aus Tumor-cDNA (Mucin1, Her2/neu, Telomerase), oder sie wurden in bakterielle Vektoren kloniert (Influenza Matrix und MAGE-A1). Die PCR-Fragmente werden mit Restriktionsenzymen geschnitten (Mucin1: BglII-SpeI; Her-2/neu: HinDIIIblunt-SpeI; Telomerase: BgIII-SpeI; MAGE-A1: BamHI-SpeI; Influenza Matrix Protein: BglII-SpeI) und in das T7TS-Plasmid (vgl. Fig. 8) über die BglII und SpeI Restriktionsstellen kloniert. Plasmide hoher Reinheit werden über das Endo-free Maxipreparation Kit (Qiagen, Hilden, Deutschland) erhalten. Die Sequenz des Vektors wird über eine Doppelstrang-Sequenzierung vom T7 Promotor bis zur PstI Stelle kontrolliert und dokumentiert. Plasmide, deren insertierte Gensequenz korrekt und ohne Mutationen ist, werden für die *In vitro* -Transkription benutzt. (Kontrolle über die publizierten Sequenzen: Accession Nummern: M11730 für Her-2/neu, NM_002456 für MUC1, NM_003219 für Telomerase TERT, V01099 für Influenza Matrix und M77481 für MAGE-A1).

### In vitro - Transkription

### Produktion von linearer, proteinfreier DNA

500 µg von jedem Phsmid werden in einem Volumen von 0,8 ml über ein Verdau mit dem Restriktionsenzym PstI in einem 2 ml Eppendorf-Reaktionsgefäß linearisiert. Dieses geschnittene Konstrukt wird in die RNA-Produktionseinheit überführt. 1 ml eines Gemischs von Phenol/Chloroform/Isoamylalkohol wird zu der linearisierten DNA gegeben Das Reaktionsgefäß wird für 2 Minuten gevortext und für 3 Minuten bei 15.000 UpM abzentrifuguiert.

Die wässerige Phase wird abgenommen und mit 0,7 ml 2-Propanol in einem 2 ml Reaktionsgefäß vermischt Dieses Gefäß wird 15 Minuten bei 15000 UpM zentrifugiert, der Überstand verworfen, und es wird 1 ml 75% Ethanol hinzugefügt. Das Reaktionsgefäß wird für 10 Minuten bei 15.000 UpM zenrifugiert, und der Ethanol wird entfernt. Das Gefäß wird nochmals für 2 Minuten zentrifugiert, und die Reste des Ethanols werden mit einer MikroliterPipettenspitze entfernt. Das DNA-Pellet wird dann in 1 µg/ml in RNase-freiem Wasser aufgelöst.

### Enzymatische Synthese der RNA

In einem 50 ml Falcon-Röhrchen wird folgendes Reaktionsgemisch hergestellt: 100 µg linearisierte proteinfreie DNA, 1 ml 5x Puffer (200mM Tris-HCl (pH7,9), 30 mM MgCl₂, 10 mM Spermidin, 50 mM NaCl, 50 mM DTI), 2001 Ribonuclease (RNase)-Inhibitor (rekombinant, 5 000 U), 1 ml rNTP-Mix (jeweils 10 mM ATP, CTP, UTP; 2 mM GTP), 1 ml CAP Analogon (8 mM), 150 µl T7 Polymerase (3000 U) und 2,55 ml RNase-freies Wasser. Das Gesamtvolumen beträgt 5 ml. Das Gemisch wird für 2 Stunden bei 37 °C im Heizblock inkubiert. Danach werden 100 U RNase-freie DNase zugefügt, und das Gemisch wird wieder für 30 Minuten bei 37 °C inkubiert. Herbei wird die DNA-Matrize enzymatisch abgebaut.

### Beschreibung und Herkunft der einzelnen Komponenten

T7 Polymerase: aufgereinigt aus einem *E. coli* -Stamm, der ein Plasmid mit dem Gen für die Polymerase enthält. Diese RNA-Polymerase verwendet als Substrat nur Promotorsequenzen des T7-Phagen; Fa. Fermentas.
NTPs: chemisch synthetisiert und über HPLC aufgereinigt. Reinheit über 96%; Fa. Fermentas.
CAP Analogon: chemisch synthetisiert und über HPLC aufgereinigt. Reinheit über 90%; Institut für Organische Chemie der Universität Tübingen.
RNase Inhibitor: RNasin, zur Injektion, rekombinant hergestellt (*E. coli*); Fa. Promega.
DNase: Pulmozym^{•} ("domase alfa"); Fa. Roche

### Reinigung

Die mit DNase behandelte RNA wird mit 20 ml von einer Lösung aus 3,3 ml 5 M NH4OAc plus 16,65 ml Ethanol vermischt Das Gemisch wird für 1 Stunde bei -20°C inkubiert und bei 4000 UpM für 1 Stunde zentrifugiert. Der Überstand wird entfernt und das Pellet mit 5 ml 75% RNase-freiem Ethanol gewaschen. Das Gefäß wird nochmals bei 4000 UpM für 15 Minuten zentrifugiert und der Überstand entfernt. Das Gefäß wird nochmals unter den vorherigen Bedingungen zentrifugiert und der verbleibende Ethanol mit einer Mikroliterpipettenspitze entfernt. Das Reaktionsgefäß wird geöffnet, und das Pellet wird unter einer Sterilbank im sterilen Umfeld getrocknet.

1 ml RNase-freies Wasser wird zur getrockneten RNA hinzugefügt. Das Pellet wird bei 4 °C für mindestens 4 Stunden inkubiert. 2 µl der wässerigen Lösung werden einer quantitativen Analyse (Bestimmung der UV-Absorption bei 260 nm) unterzogen. 2 ml einer Phenol/Chloroform/Isoamylalkohol-Lösing werden zu 1 ml wässeriger RNA-Lösung hinzugefügt. Das Gemisch wird für 2 Minuten gevortext und bei 4000 UpM für 2 Minuten zentrifugiert. Die wässerige Phase wird mit einer Mikroliterpipette entfernt und in ein neues Reaktionsgefäß überführt 4 ml einer Lösung aus 0,66 ml 5 M NH4OAc plus 3,33 ml Ethanol werden hinzugefügt. Das Gemisch wird für 1 Stunde bei -20°C inkubiert und bei 4000 UpM für 1 Stunde zentrifugiert. Der Überstand wird entfernt und das Pellet mit 75% RNase freiem Ethanol gewaschen. Das Gefäß wird nochmals bei 4000 UpM für 15 Minuten zentrifugiert, und der Überstand wird entfernt. Das Gefäß wird nochmals unter den vorherigen Bedingungen zentrifugiert und der verbleibende Ethanol mit einer Mikroliterpipettenspitze entfernt. Das Reaktionsgefäß wird geöffnet und unter einer Sterilbank das Pellet im sterilen Umfeld getrocknet.

Die RNA wird in RNase-freiem Wasser gelöst und auf eine Konzentration von 10 mg/ml eingestellt. Sie wird 12 Stunden bei 4 °C inkubiert. Durch Zugabe von Injektionspuffer (150 mM NaCl, 10 mM HEPES) wird eine Endkonzentration von 2 mg/ml erreicht. Das Endprodukt wird vorzugsweise unter GMP-Bedingungen vor Gebrauch sterilfiltriert.

### Applikation der RNA

Jeder Patient erhält an zwei verschiedenen Stellen eine intradermale (i.d.) Injektion von je 150 *µ*l der Injektionslösung, in der je 100 *µ*g Antigen-codierende mRNA (CEA, Her-2/neu, MA-GE-A1, Mucin 1, Telomerase, Influenza Matrix Protein) gelöst vorliegt.

Nach der Primärimmunisierung wird alle 14 Tage eine Booster-Immunisierung durchgeführt, um dann die Impfungen in monatlichem Abstand zu wiederholen. Jeweils einen Tag nach der RNA-Injektion wird den Patienten zusätzlich GM-CSF (Leucomax^{®}, Sandoz/Essex Pharma) sub cutan (s.c.) verabreicht.

Bei vorhandenem klinischen Ansprechen bzw. Stabilisierung der Erkrankung wird diese Therapie in monatlichen Abständen fortgeführt.

### Weitere immunologische Untersuchungen in vitro (optional)

Durchflusscytometrische Untersuchungen von PBMC zur Quantifizierung von CTL-Vorläufern;
⁵¹Cr-Freisetzungstests;
Lösliche Rezeptoren- und Cytokinspiegel im Serum;
DTH-Reaktion (Hautreaktion auf intra dermal injizierte RNA, "delayed type hypersensitivity", T-Lymphocyten vermittelte Reaktion); und
Hautbiopsieproben aus der Injektionsstelle zur histologischen Untersuchung auf T-Zell-Infiltration (Pathologie).

### Parameter zur Beurteilung der Wirksamkeit

Um die Frage nach der Wirksamkeit dieser Immuntherapie beantworten zu können, wird die Induktion tumorspezifischer T-Zellen und einer meßbaren Tumorremission herangezogen. Als Parameter gelten *in vitro* und *in vivo* gemessene T-Zellreaktionen sowie Größenänderungen bidimensional erfaßbarer Tumormanifestationen oder laborchemischer Verlaufsparameter.

Die objektive Remission ist definiert als bestes Ansprechen in Form einer kompletten oder partiellen Remission, entsprechend den unten aufgeführten Kriterien. Die Remissionsrate berechnet sich aus dem Verhältnis aus der Anzahl der Patienten mit objektiver Remission und der Gesamtzahl auswertbarer Patienten.

Als immunologisches Ansprechen auf die Therapie wird eine Veränderung im Immunstatus, bestimmt durch Immuntypisierung peripherer mononukleärer Zellen, Erhöhung der antigen-spezifischen CTL-Vorläuferfrequenz im peripheren Blut und die Induktion einer anhaltenden tumorspezifischen T-Zellaktivität gewertet. Hierzu werden *in vitro* Induktionskulturen zur Aktivierung tumorspezifischer CTL etabliert.

### Remissionskriterien (gem UICC)

| | |
|---|---|
| Komplette Remission (CR): | Vollständige Rückbildung aller messbaren Tumormanifestationen, dokumentiert durch 2 mindestens 4 Wochen auseinanderliegende Kontrolluntersuchungen. |
| Partielle Remission (PR): | Großenabnahme der Summe der Flächenmaße (Produkt zweier Tumordurchmesser oder lineare Messung eindimensional messbarer Läsionen aller Tumorbefunde um 50 % für mindestens 4 Wochen). Kein Neuauftreten von Tumormanifestationen oder Progression eines Tumorbefunds. |
| "No Change" (NC): | Abnahme aller messbaren Tumormanifestationen um weniger als 50% oder Zunahme eines Tumorbefundes. |
| Progression (PD): | Größenzunahme der Tumorparameter in mindestens einem Herd oder Neuauftreten einer Tumormanifestation. |

### Referenzen

1. Rammensee HG, Falk K, Rotzschke O: Peptides naturally presented by MHC class I molecules. Annu Rev Immunol 11: 213, 1993.
2. Bevan MJ: Antigen presentation to cytotoxic T lymphocytes in vivo. J Exp Med 182: 639,1995.
3. Rock K.L: A new foreign policy: MHC class I molecules police the outside world. Immunol Today 17:131,1996.
4. Steinman, AM: The dendritic cell system and its role in immunogenicity. Annu. Rev Immunol 9:271, 1991.
5. Steinman RM, Witmer-Pack M, Inaba K: Dendritic cells: antigen presentation, accessory function and clinical relevance. Adv Exp Med Biol 329:1, 1993.
6. Inaba K, Metlay JP, Crowley MT, Steinman RM: Dendritic cells pulsed with protein antigens in vitro can prime antigen-specific, MHC-restricted T cells in situ. J Exp Med 172:631,1990.
7. Austyn JM: New insight into the mobilisation and phagocytic activity of dendritic cells. J Exp Med 183:1287,1996.
8. Romani N, Koide S, Crowley M, Witmer-Pack M, Livingstone AM, Fathman OG, Steinman RM: Presentation of exogenous protein antigens by dendritic cells to T cell clones. J Exp Med 169:1169,1989.
9. Nair S, Zhou F, Reddy R, Huang L, Rouse BT: Soluble proteins delivered to dendritic cells via pH-sensitive liposomes induce primary cytotoxic T lymphocyte responses in vitro. J Exp Med 175:609, 1992.
10. Cohen PJ, Cohen PA, Rosenberg SA, Katz SI, Mule JJ: Murine epidermal Langerhans cells and splenic dendritic cells present tumor-associated antigens to primed T cells. Eur J Immunol 24:315, 1994.
11. Porgador A, Gilboa E: Bone-marrow-generated dendritic cells pulsed with a class I-restricted peptide are potent inducers of cytotoxic T lymphocytes. J Exp Med 182:255, 1995.
12. Celluzzi CM, Mayordomo JI, Storkus WJ, M. T. Lotze MT, and L D. Falo LD: Peptide-pulsed dendritic cells induce antigen-specific, CTL-mediated protective tumor immunity. J Exp Med 183:283,1996.
13. Zitvogel L, Mayordomo JI, Tjandrawan T, DeLeo AB, Clarke MR, Lotze MT, Storkus WJ: Therapy of murine tumors with tumor peptide-pulsed dendritic cells: dependence on T cells, B7 costimulation, and T helper cell 1-associated cytokines. J Exp Med 183:87, 1996.
14. Porgador A, Snyder D, Gilboa E: Induction of antitumor immunity using bone marrow-generated dendritic cells. J Immunol 156:2918,1996.
15. Paglia P, Chiodoni C, Rodolfo M, Colombo MP: Murine dendritic cells loaded in vitro with soluble protein prime cytotoxic T lymphocytes against tumor antigen in vivo. J Exp Med 183:317,1996.
16. Brossart P, Goldrath AW, Butz EA, Martin S, Bevan MJ: Adenovirus mediated delivery of antigenic epitopes into DC by a means of CTL induction. J Immunol 158: 3270, 1997.
17. Fisch P, Köhler G, Garbe A, Herbst B, Wider D, Kohler H, Schaefer HE, Mertelsmann R, Brugger W, Kanz L: Generation of antigen-presenting cells for soluble protein antigens ex vivo from peripheral blood CD34+hematopoetic progenitor cells in cancer patients. Eur J Immunol 26:595, 1996.
18. Sallusto F, Cella M, Danieli C, Lanzavecchia A: Dendritic cells use macropinocytosis and the mannose receptor to concentrate macromolecules in the Major Histocompatibility Complex class II compartment: Down regulation by cytokines and bacterial products. J Exp Med 182:389,1995.
19. Bernhard H, Disis ML, Heimfeld S, Hand S, Gralow JR, Cheever MA: Generetion of immunostimulatorry dendritic cells from human CD34+ hematopoetic progenitor cells of th bone marrow and periphereal blood. Cancer Res 55:1099,1995.
20. Hsu FJ, Benike C, Fagnoni F, Liles TM, Czerwinski D, Taidi B, Engelman EG, Levy R: Vaccination of patients with B-cell lympnoma using autologous anugen-pulsed dendritic cells. Nat Med 2: 52,1996.
21. Robbins PF, Kawakami Y: Human tumor antigens recognized by T cells. Curr Opin Immunol 8: 628,1996.
22. Linehan DC, Goedegebuure PS, Peoples GE, Rogers SO, Eberlein TJ: Tumor-specific and HLA-A2 restricted cytolysis by tumor-associated lymphocytes in human metastatic breast cancer. J Immunol 155: 4486,1995.
23. Peoples GE, Goedegebuure PS, Smith R, Linehan DC, Yoshino I, Eberlein TJ: Breast and ovarian cancer specific cytotoxic T lymphocytes recognize the same HER-2/-neu derived peptide. Proc Natl Acad Sci USA 92: 432,1995.
24. Fisk B, Blevins TL, Wharton JT, Ioannides CG: Identification of an immunodomonant peptide of HER-2/neu protooncogene recognized by ovarian tumor-specific cytotoxic t lymphocyte lines. J Exp Med 181: 2109, 1995.
25. Brossart P, Stuhler G, Flad T, Stevanovic S, Rammensee H-G, Kauz L and Brugger W. HER-2/neu derived peptides are tumor-associated antigens expressed by human renal cell and colon carcinoma lines and are recognized by in vitro induced specific cytotoxic T lymphocytes. Cancer Res. 58: 732-736,1998.
26. Apostolopoulos, V. and McKenzie, I. F. C, Cellular mucins: targets for immunotherapy. Crit. Rev. Immunol. 14: 293-302,1995.
27. Brossart P, Heinrich KS, Stevanovic S, Stuhler G, Behnke L, Reichardt VL, Muhm A, Rammensee H-G, Kanz L, Brugger W. Identification of HLA-A2 restricted T cell epitopes derived from the MUC1 tumor antigen for broadly applicable cancer vaccines. Blood 93: 4309-4317,1999
28. Brossart P, Wirths S, Stuhler G, Reichardt VL, Kanz L, Brugger W. Induction of CTL responses in vivo after vaccinations with peptide pulsed dendritic cells. Blood 96:3102-8, 2000
29. Kugler A, Stuhler G, Walden P, Zöller G, Zobywalski A, Brossart P, Trefzer U, Ullrich S, Müller CA, Becker V, Gross AJ, Hemmerlein B, Kanz L, Müller GA, Ringert RH. Regression of human metastatic renal cell carcinoma after vaccination with tumor celldendritic cell hybrids. Nature Med 3: 332-336, 2000 (IF 25,58)
30. Nestle FO, Alijagic S, Gilliet M, Sun Y, Grabbe S, Dummer R, Burg G, Schadendorf D (1998) Vaccination of melanoma patients with peptide- or tumor lysate-pulsed dendritic cells. Nat. Med. 4:328
31. Schuler-Thurner B, Dieckmann D, Keikavoussi P, Bender A, Maczek C, Jonuleit H, Roder C, Haendle I, Leisgang W, Dumbar R, Cerundolo V, von Den DP, Knop J, Brocker EB, Enk A, Kampgen E, Schuler G (2000) Mage-3 and influenza-matrix peptide-specific cytotoxic T cells are inducible in terminal stage HLA-A2.1+ melanoma patients by mature monocyte-derived dendritic cells. J.Immunol. 165:3492
32. Thumer B, Haendle I, Roder C, Dieckmann D, Keikavoussi P, Jonuleit H, Bender A, Maczek C, Schreiner D, von Den DP, Brocker EB, Steinman RM, Enk A, Kampgen E, Schuler G (1999) Vaccination with mage-3A1 peptide-pulsed mature, monocyte-derived dendritic cells expands specific cytotoxic T cells and induces regression of some metastases in advanced stage IV melanoma. J.Exp.Med. 190:1669

### Beispiel 5: Vakzinierung mit autologer, amplifizierter Tumor-RNA bei Patienten mit malignem Melanom

### Einleitung

Die Inzidenz des malignen Melanoms ist in den letzten Jahren weltweit stark angestiegen. Falls sich die Melanomerkrankung zum Zeitpunkt der Diagnosestellung bereits im metastasierten Stadium befindet, so gibt es derzeit keine Therapie, die den weiteren Krankheitsverlauf mit ausreichender Sicherheit positiv beeinflußt.

Bislang durchgeführte Vakzinierungstherapien unter Verwendung von dendritischen Zellen sind wegen der komplizierten Anzüchtung der Zellen sehr labor-, kosten- und zeitintensiv (GMP-Bedingungen). Weiterhin konzentrierten sich die Studien bislang vorwiegend auf bekannte tumorassoziierte Antigene (TAA), wie zum Beispiel Melan-A oder Tyrosinase.

Eine Reihe verschiedener immunologischer Phänomene wie unter anderem das Auftreten von spontanen Tumorregressionen oder der spontanen Involution von Metastasen haben das Melanom zum vorrangigen Kandidaten zur Erprobung immuntherapeutischer Untersuchungen gemacht (Parkinson et al., 1992). Neben Versuchen der unspezifischen Stimulation des Immunsystems mittels Interleukin-2, Mistelextrakten, BOG und Interferonen, die bisher zu keinen entscheidenden Durchbrüchen in der Therapie fortgeschrittener Tumorerkrankungen führten, wurde in den letzten Jahren insbesondere die Strategie der Induktion verschiedener hochspezifischer cytotoxischer T-Lymphocyten (CTL) verfolgt. Diese CTL sind in der Lage autologe Melanomzellen zu erkennen und abzutöten (Boon et aL, 1994; Houghton, 1994). Untersuchungen dieses Vorgangs ergaben, dass die CIL definierte Peptide in Verbindung mit MHC-Klasse-I-Molekülen erkennen. Die Präsentation von Peptiden durch Antigen-präsentierende Zellen (APC) sind der physiologische Weg zur Erzeugung von spezifischen Immunantworten durch Lymphocyten (Rammensee, 1993). Dendritische Zellen haben sich als potente Antigen-präsentierende Zellen erwiesen, die auf zwei Wegen zu einer Induktion der Immunantwort führen: Der erste ist die direkte Präsentation von Peptiden gegenüber CD8⁺-T-Lymphocyten und deren Aktivierung (Schuler & Steinmann, 1985; Inaba et al., 1987; Romani et al., 1989), der zweite ist die Erzeugung einer protektiven Immunantwort, die von CD4⁺-Helferlymphocyten vermittelt wird, und eine Präsentation von Peptiden über MHC-Klasse-II-Moleküle voraussetzt (Grabbe et al., 1991,1992,1995).

Mittels der Peptidanalyse konnten so verschiedene Tumor-assozierte Antigene (TAA) identifiziert werden, die für das Melanom spezifisch sind und nach Präsentation in Verbindung mit dem MHC-Molekül und Erkennung durch die CTL zur Cytolyse der Tumorzellen führen (Schadendorf et al., 1997, S. 21-27).

Die Verwendung von autologen, dendritischen Zellen wurde im Rahmen einer Pilotstudie bei Melanompatienten bezüglich seines Potentials, effektiv, schnell und zuverlässig cytotoxische T-Lymphocyten zu induzieren, getestet. In dieser Studie wurden 16 bereits chemotherapeutisch vorbehandelte Melanompatienten im Stadium IV mit Peptid-beladenen dendritischen Zellen vakziniert. Die Ansprechraten lagen über 30% (5/16 Patienten) (Nestle et al., 1998). In einer weiteren unabhängigen Studie konnte eine noch höhere Ansprechrate von mehr als 50% (6/11 Patienten) nach Immunisierung von bereits chemotherapeutisch vorbehandelten, metastasierten Melanompatienten mit MAGE-3A1-beladenen dendritischen Zellen gezeigt werden (Thumer et aL, 1999). Ebenfalls wurde eine signifikante Expansion von MAGE-A3-spezifischen CD8⁺-T-Zellen in 8/11 Patienten beobachtet. Es erfolgte nach der DG Vakzinierung in einigen Fällen eine Regression der Metastasen. Dies wurde von einer CD8⁺-T-Zell-Infiltration begleitet. Dies zeigte, dass die induzierten T-Zellen *in vivo* aktiv waren. Nachteil dieser Strategie ist der große kosten- und labortechnische Aufwand (insbesondere GMP-Bedingungen). Für die zeitintensive Generierung der DC werden große Mengen an Patientenblut benötigt. Bei der Herstellung der Peptide kann zum einen nur auf bekannte tumorassoziierte Antigene zurückgegriffen werden, zum anderen sind je nach HLA-Haplotyp verschiedene Peptide notwendig.

Eine Weiterentwicklung dieses Ansatzes ist die Vakzinierung mit RNA-transfizierten DC (Nair et al., 1998, Nair et al., 2000). Zahlreiche Studien belegen inzwischen, daß DC von Maus und Mensch, die mit mRNA transfizien wurden, eine effiziente CTL-Antwort *in vitro* und in-vivo auslösen können und zu einer deutlichen Reduktion von Metastasen führen können (Boczkowski et aL, 1996, 2000; Ashley et al, 1997; Nair et al., 1998, 2000; Heiser et al., 2001; Mitchell and Nair, 2000; Koido et al., 2000; Schmitt et aL, 2001). Ein großer Vorteil bei der Verwendung von RNA gegenüber Peptiden ist, dass verschiedenste Peptide aus einer für ein TAA codierenden mRNA prozessiert und präsentiert werden können. Durch eine derartige polyvalente Vakzine kann die Wahrscheinlichkeit für das Auftreten von sogenannten klonalen "tumor escape"- Phänomenen deutlich gesenkt werden. Darüber hinaus können durch diesen Ansatz T-Zell-vermittelte Immunantworten gegen auf natürlichem Wege prozessierte und präsentierte Antigene mit potentiell höherer Immundominanz induziert werden. Durch zusätzliche Beteiligung von MHC-Klasse-II-restringierten Epitopen kann die induzierte tumorspezifische Immunantwort verstärkt und länger aufrecht erhalten werden. Trotzdem ist auch dieses Verfahren wegen der nötigen Kultivierung der autologen DCs nur mit hohem Laboraufwand (GMP-Bedingungen) durchführbar.

Bei der vorliegenden erfindungsgemäßen Strategie wird mit dem im autologen Tumor des Patienten vorhandenen RNA-Expressionsprofil vakziniert. Dadurch wird dem spezifischen Tumorprofil des Patienten Rechnung getragen, wobei auch unbekannte TAAs in die Impfung mit eingehen. Die aufwendige Anzucht der DC entfällt, da bei der Vakzinierung RNA (keine transfizierten DCs) verwendet werden.

Erfindungsgemäß wird daher eine Impftherapie unter Verwendung von amplifizierter autologer Tumor-RNA an Patienten mit metastasiertem malignem Melanom, insbesondere Stadium III/IV, bereitgestellt.

Durch die Vakzinierung werden *in vivo* eine tumorspezifische cytotoxische T-Zellen indurziert, um so einen klinisch-therapeutischen Effekt (Tumor-Response) zu erreichen Es handelt sich um einen Immunisierungsansatz, der nur minimale Eingriffe beim Patienten (Injektion) erfordert. Die Therapie kann ambulant erfolgen und ist für viele Tumorpatienten geeignet ohne die Einschränkung auf bestimmte HLA-Typen oder definierte T-Zellepitope. Darüber hinaus können durch diese Therapie polyklonale CD4⁺ T-Helfer als auch CD8⁺-CTL induziert werden. Von der Strategie her entscheidend ist auch die Berücksichtigung bislang unbekannter TAAs beim Impfprotokoll, sowie die ausschließliche Verwendung von autologem Material besonders vorteilhaft.

### Behandlungplan

Die amplifizierte autologe Tumor-RNA wird dem Patienten i.d. an den Tagen 0,14,28 und 42 verabreicht. Zusätzlich erhalten die Patienten jeweils einen Tag nach der RNA-Impfung GM-CSF (Leucomax^{®} 100 µg/m², Novartis/Essex) s.c. Jeder Patient erhält an zwei verschiedenen Stellen eine i.d. Injektion von je 150 µl der Injektionslösung, in der je 100 µg autologe Tumor-RNA gelöst ist.

2 Wochen nach der vierten Injektion (Tag 56) werden gff. das Ansprechen des Tumors durch eine Staging-Untersuchung (u.a. Sonografie, Röntgen-Thorax, CT usw.; siehe hierzu die weiter unten stehenden Ausführungen) sowie durch die Auswertung der durch die Therapie induzierten immunologischen Parmeter evaluiert.

Bei stabilem Krankheitsverlauf oder einem objektiven Tumoransprechen (CR bzw. PR) erhalten die Patienten die Vakzinierungen alle 4 Wochen. Weitere Restaging-Untersuchungen können bspw. Tag 126, dann im 12-wöchigem Abstand vorgesehen weiden.

Der Behandlungsplan ist in der Fig. 13 schematisch dargestellt.

### Herstellung autologer Tumor-RNA

Ziel ist die Herstellung autologer Poly(A⁺)-RNA. Hierfür wird aus patienteneigenen Tumorgewebe Poly(A⁺)-RNA isoliert. Diese isolierte RNA ist an sich sehr instabil und in ihrer Menge limitiert. Deshalb wird die genetische Information in eine wesentlich stabilere cDNA-Bibliothek umeschrieben und somit konserviert. Ausgehend von der patienteneigenen cDNA-Bibliothek kann für den gesamten Behandlungszeitraum stabilisierte autologe RNA hergestellt werden. Das erfindungsgemäße Vorgehen ist in der Fig. 10 schematisch dargestellt.

### RNA-Isolierung

Zur Isolierung von Gesamt-RNA aus einer Tumorgewebe-Biopsie wird ein Verfahren der Fa. Roche AG angewendet. Hierbei wird das High Pure RNA Isolation Kit (Bestellnummer 1828665) nach Herstellerangaben eingesetzt. Poly(A⁺)-RNA wird aus der Gesamt-RNA über ein weiteres Verfahren der Roche AG mit dem High Pure RNA Tissue Kit (Bestellnummer 2033674) isoliert.

### Herstellung einer dDNA-Bibliothek

Die cDNA Bibliothek wird mit dem "SMART PCR cDNA Synthesis Kit" (Fa. Clontech Inc., USA; Bestellnummer PT3041-1) gemäß den Angaben des Herstellers konstruiert.

Hierbei wird die einzelsträngige Poly(A⁺)-RNA über einen speziellen Primer revers transkribiert. Über einen poly-G-Überhang am 3'-Ende der neu synthetisierten DNA kann ein weiterer Primer hybridisieren, über den das Konstrukt durch PCR amplifiziert werden kann. Die doppelsträngigen cDNA-Fragmente stehen nun zur Klonierung in einen geeigneten RNA-Produktionsvektor (z.B. pT7TS; vgl. Fig. 8) bereit.

Das Verfahren zur Herstellung der cDNA Bibliothek aus der Poly-A⁺-RNA mit Hilfe des obigen Kits ist in der Fig. 11 schematisch dargestellt.

### Plasmid-Konstrukte

Die cDNA-PCR-Fragmente werden mit den Restriktionsenzymen NotI und SpeI geschnitten und in die entsprechenden Restriktionsstellen des pT7TS Vektors analog der im Beispiel 4 angegebenen Vorgehensweise kloniert. Plasmide hoher Reinheit werden über das Endo-free Maxipreparation Kit (Qiagen, Hilden, Deutschland) erhalten. Plasmide, deren einklonierte Gensequenz der erwarteten Grössenfraktionierung (200bp-4000bp) der cDNA-Bibliothek entsprechen, werden für die *In vitro* -Transkription benutzt. Ein Beispiel einer Auftrennung einer repräsentativen cDNA-Billiothek in einem Agarosegel ist in der Fig. 12 gezeigt.

### In vitro-Transkription und RNA-Applikation

Die *In vitro* -Transkription und die Verabreichung der RNA erfolgen wie im vorstehenden Beispiel 4 beschrieben.

### Untersuchungen während der Behandlung

Vor jeder Impfung (am Tag der Impfung):
Körperliche Untersuchung (einschließlich RR, Fieber);
Blutabnahme Routine-Laborwerte
   1. Blutbild, Differential-Blutbild: 3 ml
   2. Elektrolyte, LDH, CK, Leberenzyme, Bilirubin, Kreatinin, Harnsäure, Gesamteiweiß, CRP: 5 ml
   3. Blutsenkung: 2 ml; und
bei Wiederholungsimpfungen zusätzlich: Inspektion der Injektionsstellen.

Am Tag 1 nach jeder Impfung:
Körperliche Untersuchung (einschließlich RR, Fieber); und
Inspektion der Injektionsstellen.

Bei Staging-Untersuchungen am Tag 56 und 126 nach der ersten Impfung, dann alle 12 Wochen, zusätzlich:
Erweiterte Routine-Blutentnahme:
   1. Tumormarker S100 *(*7 ml)
   2. Gerinnungswerte (3 ml);
Blutentnahme Immunmonitoring (30 ml);
Allgemeinbefinden (ECOG-Score);
Bildgebende Verfahren (Röntgen-Thorax, Sonographie, Skelettszintigramm CT-Abdomen, Becken, Thorax, Schädel); und
EKG.

### Weitere immunologische Untersuchungen in vitro

Es wird ggf. die relative Häufigkeit von antigen-spezifischen CTL-Vorläuferzellen im peripheren Blut des Patienten im zeitlichen Verlauf der Impftherapie gemessen.

Zum einen werden hierbei mit FACS-Analysen (Tetramerfärbung) CTL-Vorläuferzellen quantifiziert, die gegen von Melanomzellen im besonderen Maße exprimierte Antigene gerichtet sind (Tyrosinase, MAGE-3, Melan-A, GP100). Zum anderen werden ELIspot-Untersuchungen durchgeführt, die so ausgelegt sind, dass zusätzlich CTL-Vorläuferzzellen erfasst werden, die spezifisch gegen bislang unbekannte Antigene gerichtet sind. Dazu werden autologe dendritische Zellen, die aus dem peripheren Blut der Patienten kultiviert werden, mit der gleichen RNA inkubiert, mit der auch die Impfung durchgeführt wurde. Diese dienen dann als Stimulatorzellen in der ELIspot-Untersuchung. Die Messung erfasst somit das gesamte Impfspektrum. Für diese Untersuchungen können Blutentnahmen für das Immunmonitoring im Rahmen der Staging-Untersuchungen und zusätzlich an den Tagen 0,14, 28 und 42 von insgesamt 30 ml (20 ml ELIspot, 10 ml FACS-Analyse) vorgesehen werden, sowie eine einmalige Abnahme von 100 ml an Tag 70 zur Anzucht der DC.

Des Weiteren können Hautbiopsieproben aus der Injektionsstelle zur histologischen Untersuchung hinsichtlich einer T-Zell-Infiltration gewonnen weiden.

### Parameter zur Beurteilung der Wirksamkeit

Die Wirksamkeit der erfindungsgemäßen Therapie wird anhand der vorstehend im Beispiel 4 angegebenen Parameter bewertet.

### Referenzen

Anichini, A, Mortarini, R., Maccalli, C., Squarcina, P., Fleishhauer, K., Mascheroni, L, Parmiani, G. (1996). Cytotoxic T cells directed to tumor antigens not expressed on normal melanocytes dominate HLA-A2.1-restricted immune repertoire to melanoma. J. ImmunoL 156, 208-217.
Ashley, DM, Faiola, B., Nair, S., Hale, LP., Bigner, DD, Gilboa, E. (1997). Bone marrow-generated dendritic cells pulsed with tumor extracts or tumor RNA induce anti-tumor immunity against central nervous system tumors. J. Exp. Med. 186, 1177-1182.
Boczkowski, D., Nair, SK., Synder, D., Gilboa, E. (1996). Dendritic cells pulsed with RNA are potent antigen-presenting cells in vitro and in vivo. J. Exp. Med. 184, 465-472.
Boczkowski, D., Nair, SK., Nam, J., Lyerly, K., Gilboa, E. (2000). Induction of tumor immunity and cytotoxic T lymphocyte responses using dendritic cells transfected with messenger RNA amplified from tumor cells. Cancer Res. 60, 1028-1034.
Boon, T., Coulie, P., Marchand, M., Weynants, V. (1994). Genes coding for tumor rejection antigens: perspectives for specific immunotherapy. In Important Advances in Oncology 1994. De Vita, VT, Hellman, S., Rosenberg, SA, ed. (Philadelphia: Lippincott Co), pp. 53-69.
Garbe, C, Orfanos, CE (1989): Epidemiologie des malignen Melanoms in der Bundesrepublik Deutschland im internationalen Vergleich. Onkologie 12,253-262.
Grabbe, S., Bruvers, S., Gallo, R.L., Knisely, T.L., Nazareno, R., and Granstein, RD. (1991). Tumor antigen presentation bymurine epidermal cells. J Immunol. 146, 3656-3661.
Grabbe, S., Bruvers, S., Lindgren, A.M., Hosoi, J., Tan, K.C., and Granstein, R.D. (1992). Tumor antigen presentation by epidermal antigen-presenting cells in the mouse: modulation by granulocyte-macrophage colony-stimulating factor, tumor necrosis factor alpha, and ultraviolet radiation. J Leukoc.Biol. 52, 209-217.
Grabbe, S., Beissert, S., Schwarz, T., and Granstein, R.D. (1995). Dendritic cells as initiators of tumor immune responses: a possible strategy for tumor immunotherapy?. Immunol. Today 16, 117-121.
Grünebach, F, Müller, MR, Nencioni, A, Brugger, W, and Brossart, P (2002). Transfection of dendritic cells with RNA induces cytotoxic T lymphocytes against breast and renal cell carcinomas and reveals the immunodominacnce of presented T cell epitopes. submitted.
Heiser, A., Maurice, MA., Yancey, DR., Coleman, DM, Dahm, P., Vieweg, J. (2001). Human dendritic cells transfected with renal tumor RNA stimulate polyclonal T cell responses against antigens expressed by primary and metastaric tumors. Cancer Res. 61, 3388-3393.
Heiser, A., Maurice, MA., Yancey, DR., Wu, NZ., Dahm, P., Pruitt, SK., Boczkowski, D., Nair, SK., Ballo, MS., Gilboa, E., Vieweg, J. (2001). Induction of polyclonal prostate cancer-specific CTL using dendritic cells transfected with amplified tumor RNA. J. Immunol. 166, 2953-2960.
Hoerr, I, Obst, R, Rammensee, HG, Jung, G (2000). In vivo application of RNA leads to induction of specific cytotoxic T lymphocytes and antibodies. Eur J Immunol. 30,1-7.
Hougbton, AN (1994). Cancer antigens: immune recognition of self and ahered self. J.Exp.Med 180, 1-4
Inaba, K, Young, JW and Steinman, RM (1987). Direct activation of CD8+ cytotoxic T lymphocytes by dendritic cells. J.Exp.Med. 166, 182-194.
Koido, S., Kashiwaba, M, Chen, D., Gendler, S., Kufe, D., Gong, J. (2000). Induction of antitumor immunity by vaccination of dendritic cells transfected with MUC1 RNA. J. Immunol. 165, 5713-5719.
Mitchell, DA, Nair, SK. (2000). RNA-transfected dendritic cells in cancer immunotherapy. J. Clin. Invest. 106, 1065-1069.
Nair, S., Boczkowski, S., Synder, D., Gilboa, E. (1998). Antigen presenting cells pulsed with unfractionated tumor-derived peptides are potent tumor vaccines. Eur. J. Immunol. 27, 589-597.
Nair, S., Heiser, A., Boczkowski, D., Majumdar, A., Naoe, M., Lebkowski, JS., Vieweg, J., Gilboa, E. (2000). Induction of cytotoxic T cell responses and tumor immunity against unrelated tumors using telomerase reverse transcriptase RNA transfected dendritic cells. Nat. Med. 6,1011-1017.
Nestle, F.O., Alijagic, S., Gilliet, M., Sun, Y., Grabbe, S., Dummer, R, Burg, G., and Schadendorf, D. (1998). Vaccination of melanoma patients with peptide- or tumor lysate-pulsed dendritic cells. Nat.Med 4, 328-332.
Parkinson, DR, Houghton, AN, Hersey, P, Borden, EC (1992). Biologic therapy for melanoma. I Cutaneous melanoma. Balch, CM, Houghton, AN, Milton GW, Soober, AJ, Soong, SJ, ed. (Lippincott Co), pp. 522-541
Rammensee, H.G., Falk, K., and Rotzschke, O. (1993). Peptides naturally presented by MHC class I molecules. Annu.Rev.Immunol. 11, 213-244.
Romani N, Koide S, Crowley M, Witmer-Pack M, Livingstone AM, Fathman CG, Steinman RM: Presentation of exogenous protein antigens by dendritlc cells to T cell clones. J Exp Med 169:1169,1989.
Schadendorf, D, Grabbe, S, Nestle, FO (1997). Vaccination with Dendritic Cells - A specific Immunomodulatory Approach. In Strategies for Immunointervention in Dermatoilogy. Burg, G, Dummer, RG, ed. (Heidelberg, New York: Springer-Verlag),
Schmitt, WE., Stassar, MJJG., Schmitt, W., Littlee, M, Cochlovius, B. (2001). In vitro induction of a bladder cancer-specific T-cell response by mRNA-transfected dendritic cells. J. Cancer Res. Clin. Oncol. 127, 203-206.
Schmoll HJ, Höffken K, Possinger K (1997): Kompendium Internistische Onkologie, 2. Aufl., Springer-Verlag Berlin, Teil 2,1415.
Schuler G and Steinmann RM (1985). Murine epidermal Langerhans cells mature into potent immunostimulatory dendritic cells in vitro. J. Exp. Med. 161, 526-546.
Thumer, B., Haendle, I., Roder, C. et aL (1999). Vaccination with Mage-3A1 peptide-pulsed mature, monocyte-derived dendritic cells expands specific cytotoxic T cells and induces regression of some metastases in advanced stage IV melanoma. J. Exp. Med. 190, 1669-1678.

## Patentansprüche

1. Verfahren zur Herstellung einer pharmazeutischen Zusammensetzung, umfassend die Schritte:
(a) Herstellen eines Teils einer cDNA-Bibliothek aus Tumorgewebe eines Patienten, wobei der Teil eine Subtraktionsbibliothek der Gesamt-cDNA-Bibliothek darstellt und für tumorspezifische Antigene codiert, vor Schritt (a) die Sequenzen der tumorspezifischen Antigene ermittelt werden und wobei das Ermitteln der Sequenzen der tumorspezifischen Antigene einen Abgleich mit einer cDNA-Bibliothek aus gesundem Gewebe umfasst,
(b) Herstellen einer Matrize für die *In vitro* -Transkription von RNA anhand des Teils der cDNA-Bibliothek und
(c) *In vitro* -Transkribieren der Matrize.

2. Verfahren nach Anspruch 1, wobei für die pharmazeutische Zusammensetzung das oder die Antigen(e) aus einem Tumor aus der Gruppe, bestehend aus 707-AP, AFP, ART-4, BAGE, β-Catenin/m, Bcr-abl, CAMEL, CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A*0201-R170I, HPV-E7, HSP70-2M, HAST-2, hTERT (oder hTRT), iCE, KIAA0205, LAGE, LDLR/FUT, MAGE, MART-1/Melan-A, MC1R, Myosin/m, MUC1, MUM-1, -2, -3, NA88-A, NY-ESO-1, p190 minor bcr-abl, Pml/RARα, PRAME, PSA, PSM, RAGE, RU1 oder RU2, SAGE, SART-1 oder SART-3, TEL/AML1, TPI/m, TRP-1, TRP-2, TRP-2/INT2 und WT1, transkribiert wird/werden.

3. Verfahren nach Anspruch 1 oder 2, wobei in der pharmazeutische Zusammensetzung mRNA-Moleküle mit einer 5'-Cap-Struktur und/oder einem Poly(A*)-Schwanz von mindestens 25 Nucleotiden und/oder mit mindestens einer IRES und/oder mit mindestens einer 5'-Stabilisierungssequenz und/oder mit mindestens einer 3'-Stabilisierungssequenz bereitgestellt werden.

4. Verfahren nach einem der vorangegangenen Ansprüche 1 bis 3, wobei der pharmazeutischen Zusammensetzung weiter ein oder mehrere Adjuvanzien zugegeben werden.

5. Verfahren nach Anspruch 4, wobei der pharmazeutischen Zusammensetzung ein Adjuvans ausgewählt aus der Gruppe, bestehend aus Lipopolysaccharid, TNF-α, CD40-Ligand, GP96, Oligonucleotiden mit dem CpG-Motiv, Aluminiumhydroxid, Freudsches Adjuvans, Lipopeptiden und Cytokinen, zugegeben wird.

6. Verfahren nach Anspruch 5, wobei der pharmazeutischen Zusammensetzung das Cytokin GM-CSF zugegeben wird.

7. Verfahren nach einem der vorangehenden Ansprüche 1 bis 6, wobei in der pharmazeutischen Zusammensetzung die mRNA-Moleküle mit mindestens einem kationischen oder polykationischen Agens komplexiert oder kondensiert werden.

8. Verfahren nach Anspruch 7, wobei das kationische oder polykationische Agens aus der Gruppe, bestehend aus Protamin, Poly-L-Lysin, Poly-L-Arginin und Histonen, ausgewählt ist.

9. Verfahren nach einem der vorangehenden Ansprüche 1 bis 8, wobei der pharmazeutischen Zusammensetzung weiter mindestens ein RNase-Inhibitor zugegeben wird.

10. Verfahren nach Anspruch 9, wobei der RNase-Inhibitor RNasin ist.

11. Verfahren nach einem der vorangehenden Ansprüche 1 bis 10, wobei mindestens ein weiterer pharmazeutisch verträglicher Träger und/oder mindestens ein weiteres pharmazeutisch verträgliches Vehikel zugegeben wird.

12. Verfahren nach einem der Ansprüche 1 bis 11, wobei der Abgleich einen Vergleich der Expressionsmuster von gesundem und Tumorgewebe umfasst.

13. Verfahren nach Anspruch 12, wobei das Ermitteln der Sequenzen der tumorspezifischen Antigene eine Diagnose durch einen Mikroarray umfasst.

14. Verfahren nach einem der Ansprüche 12 oder 13, wobei die Expressionsmuster auf Nukleinsäureebene verglichen werden.

15. Verfahren nach Anspruch 12, wobei die Expressionsmuster durch Expressionsanalyse auf Proteinebene bestimmt werden.

16. Pharmazeutische Zusammensetzung erhältlich nach irgendeinem der Ansprüche 1 bis 15.

## Claims

1. Process for the preparation of a pharmaceutical composition comprising the steps:
(a) preparation of a part of a cDNA library from tumour tissue of a patient, wherein the part is a subtraction library of the entire cDNA library and codes for tumour-specific antigens, the sequences of the tumour specific antigens are determined before step (a) and wherein the determination of the sequences of the tumour-specific antigens comprises an alignment with a cDNA library from healthy tissue.
(b) preparation of a matrix for *in vitro* transcription of RNA with the aid of the part of the cDNA library and
(c) *in vitro* transcribing of the matrix.

2. Process according to claim 1, wherein, for the pharmaceutical composition, the antigen(s) from a tumour is/are transcribed from the group consisting of 707-AP, AFP, ART-4, BAGE, β-catenin/m, Bcr-abl, CAMEL, CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A*0201-R170I, HPV-E7, HSP70-2M, HAST-2, hTERT (or hTRT), iCE, KIAA0205, LAGE, LDLR/FUT, MAGE, MART-1/melan-A, MC1R, myosin/m, MUC1, MUM-1, -2, -3, NA88-A, NY-ESO-1, p190 minor bcr-abl, Pml/RARα, PRAME, PSA, PSM, RAGE, RU1 or RU2, SAGE, SART-1 or SART-3, TEL/AML1, TPI/m, TRP-1, TRP-2, TRP-2/INT2 and WT1.

3. Process according to claim 1 or 2, wherein, in the pharmaceutical composition, the mRNA molecules are provided with a 5' cap structure and/or a poly(A⁺) tail of at least about 25 nucleotides and/or at least one IRES and/or at least one 5'-stabilizing sequence and/or at least one 3'-stabilizing sequence.

4. Process according to any of the proceeding claims 1 to 3, wherein one or more adjuvants are further added to the pharmaceutical composition.

5. Process according to claim 4, wherein an adjuvant is added to the pharmaceutical composition selected from the group consisting of lipopolysaccharide, TNF-α, CD40 ligand, GP96, oligonucleotides with the CpG motif, aluminium hydroxide, Freund's adjuvant, lipopeptides and cytokines.

6. Process according to claim 5, wherein the cytokine GM-CSF is added to the pharmaceutical composition.

7. Process according to any of the proceeding claims 1 to 6, wherein, in the pharmaceutical composition, the mRNA molecules are complexed or condensed with at least one cationic or polycationic agent.

8. Process according to claim 7, wherein the cationic or polycationic agent is selected from the group consisting of protamine, poly-L-lysine, poly-L-arginine and histones.

9. Process according to any of the proceeding claims 1 to 8, wherein at least one RNase inhibitor is further added to the pharmaceutical composition.

10. Process according to claim 9, wherein the RNase inhibitor is RNasin.

11. Process according to any of the proceeding claims 1 to 10, wherein at least one further pharmaceutically acceptable carrier and/or at least one further pharmaceutically acceptable vehicle is added.

12. Process according to any of claims 1 to 11, wherein the alignment comprises a comparison of the expression patterns of healthy and tumour tissue.

13. Process according to claim 12, wherein the determination of the sequences of the tumour-specific antigens comprises a diagnosis by a microarray.

14. Process according to any of claims 12 or 13, wherein the expression patterns are compared at the nucleic acid level.

15. Process according to claim 12, wherein the expression patterns are determined by expression analysis at the protein level.

16. Pharmaceutical composition obtainable according to any of claims 1 to 15.

## Revendications

1. Procédé de fabrication d'une composition pharmaceutique, comprenant les étapes suivantes :
(a) fabrication d'une partie d'une bibliothèque d'ADNc à partir de tissus tumoraux d'un patient, cette partie représentant un sous-ensemble de la bibliothèque d'ADNc totale et codant pour des antigènes tumoraux spécifiques, les séquences des antigènes tumoraux spécifiques étant obtenues avant l'étape (a) et l'obtention des séquences des antigènes tumoraux spécifiques comprenant un alignement avec une bibliothèque d'ADNc de tissus sains,
(b) fabrication d'une matrice pour la transcription *in vitro* d'ARN en présence d'une partie de la bibliothèque d'ADNc et
(c) transription *in vitro* de la matrice.

2. Procédé selon la revendication 1, dans lequel l'antigène ou les antigènes issus d'une tumeur du groupe constitué de 707-AP, AFP, ART-4, BAGE, β-caténine/m, Bcr-abl, CAMEL; CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A*0201-R1701, HPV-E7, HSP70-2M, HAST-2, hTERT (ou hTRT), iCE, KIAA0205, LAGE, LDLR/FUT, MAGE, MART-1/Melan-A, MC1R, myosine/m, MUC1, MUM-1, -2, -3, NA88-A, NY-ESO-1, p190 mineur, bcr-abl, Pm1/RARα, PRAME, PSA, PSM, RAGE, RU1 ou RU2, SAGE, SART-1 ou SART-3, TEL/AML1, TPI/m, TRP-1, TRP-2, TRP-2/INT2 et WT1 sont transcrits dans la composition pharmaceutique.

3. Procédé selon la revendication 1 ou 2, dans laquelle on propose dans la composition pharmaceutique des molécules d'ARNm avec une structure Cap en 5' et/ou une queue poly(A+) d'au moins 25 nucléotides et/ou avec au moins un IRES et/ou une séquence de stabilisation en 5' et/ou avec au moins une séquence de stabilisation en 3'.

4. Procédé selon l'une des revendications 1 à 3, dans lequel un ou plusieurs adjuvants sont ajoutés à la composition pharmaceutique.

5. Procédé selon la revendication 4, dans lequel un adjuvant choisi dans le groupe constitué de liposaccharide, TNF-α, ligand CD40, GP96, les oligonucléotides avec un motif CpG, l'hydroxyde d'aluminium, un adjuvant de Freud, des lipopeptides et des cytokines, est ajouté à la composition pharmaceutique.

6. Procédé selon la revendication 5, dans lequel la cytokine GM-CSF est ajoutée à la composition pharmaceutique.

7. Procédé selon l'une des revendications 1 à 6, dans lequel les molécules d'ARNm sont condensées ou complexées dans la composition pharmaceutique avec au moins un agent cationique ou polycationique.

8. Procédé selon la revendication 7, dans lequel l'agent cationique ou polycationique est choisi dans le groupe constitué de la protamine, de la poly-L-lysine, de la poly-L-arginine et des histones.

9. Procédé selon l'une des revendications 1 à 8, dans lequel au moins un inhibiteur de la RNase est ajouté en plus à la composition pharmaceutique.

10. Procédé selon la revendication 9, dans lequel l'inhibiteur de la RNase est la RNasine.

11. Procédé selon l'une des revendications 1 à 10, dans lequel au moins un autre support pharmaceutiquement accepté et/ou au moins un autre véhicule pharmaceutiquement accepté est ajouté.

12. Procédé selon l'une des revendications 1 à 11, dans lequel l'alignement comprend la comparaison de modèles d'expression de tissus sains et tumoraux.

13. Procédé selon la revendication 12, dans lequel l'obtention des séquences des antigènes tumoraux spécifiques comprend un diagnostic grâce à une micropuce.

14. Procédé selon l'une des revendications 12 ou 13, dans lequel les modèles d'expression sont comparés au niveau des acides nucléiques.

15. Procédé selon la revendication 12, dans lequel le modèle d'expression est déterminé par analyse de l'expression au niveau des protéines.

16. Composition pharmaceutique susceptible d'être obtenue par le procédé selon l'une des revendications 1 à 15.
